# EUROPÄISCHE PATENTSCHRIFT

(11) **EP 3 011 183 B1**
(45) Veröffentlichungstag und Bekanntmachung des Hinweises auf die Patenterteilung: **18.03.2020**
(21) Anmeldenummer: 14735467.4
(22) Anmeldetag: 20.06.2014
(51) Int. Cl.: F04D 3/00, F04D 13/06, F04D 29/02, F04D 29/52

(54) **PUMPENGEHÄUSE MIT HARTER INNENSCHICHT UND VERSCHWEISSBARER AUSSENSCHICHT**
PUMP HOUSING WITH HARD INNER LAYER AND WELDABLE OUTER LAYER
CORPS DE POMPE POURVU D'UNE COUCHE INTERNE DURE ET D'UNE COUCHE EXTERNE SOUDABLE

(30) Priorität: 21.06.2013 DE 102013211845
(43) Veröffentlichungstag der Anmeldung: 27.04.2016
(73) Patentinhaber: Heraeus Deutschland GmbH & Co. KG, 63450 Hanau (DE)
(72) Erfinder: SCHIBLI, Stefan, 60326 Frankfurt (DE); TRÖTZSCHEL, Jens, 63549 Ronneburg (DE)
(74) Vertreter: Herzog IP Patentanwalts GmbH
(86) Internationale Anmeldenummer: PCT/EP2014/001685
(87) Internationale Veröffentlichungsnummer: WO 2014/202226

(56) Entgegenhaltungen:
- EP-A1- 2 236 229
- DE-U1- 29 723 409
- US-A- 4 603 062
- US-B1- 6 234 772

## Beschreibung

Die Erfindung betrifft eine Pumpvorrichtung, beinhaltend i. einen Impeller; ii. ein Pumpengehäuse, das einen Innenbereich zumindest zu einem Teil umgibt, mit einem Einlass und einem Auslass, wobei der Impeller im Innenbereich des Pumpengehäuses vorgesehen ist; wobei das Pumpengehäuse einen Verbund aus einem ersten Hohlköper und einem den ersten Hohlkörper auf der dem Innenbereich abgewandten Seite zumindest zum Teil umgebenden weiteren Hohlkörper beinhaltet; wobei mindestens 60 % der Oberfläche der dem Innenraum abgewandten Seite des ersten Hohlkörpers mit dem weiteren Hohlkörper verbunden sind, bezogen auf die Oberfläche der dem Innenraum abgewandten Seite des ersten Hohlkörpers, wobei das Pumpengehäuse mit einer weiteren Oberfläche des ersten Hohlkörpers dem Innenbereich zugewandt ist; wobei die dem Innenbereich des Pumpengehäuses zugewandte weitere Oberfläche des ersten Hohlkörpers eine Härte von mindestens 330 HV aufweist, wobei der weitere Hohlkörper einen Metallgehalt von mindestens 50 Gew.-%, bezogen auf die Gesamtmasse des weiteren Hohlkörpers beinhaltet, und wobei die Pumpvorrichtung dazu geeignet ist, in den Körper eines Menschen oder eines Tieres eingebracht zu werden. Weiterhin betrifft die Erfindung ein Gehäuse mit den Merkmalen, wie zuvor für das Pumpengehäuse beschrieben.

Darüber hinaus betrifft die Erfindung ein Verfahren zur Herstellung eines Pumpengehäuses, beinhaltend die Schritte: a. Bereitstellen eines ersten Hohlkörpers; b. Bereitstellen eines weiteren Hohlkörpers, wobei mindestens der erste Hohlkörper als zylindrischer Hohlkörper ausgebildet ist; c. Kontaktieren des ersten Hohlkörpers mit dem weiteren Hohlkörper, wobei ein Verbund gebildet wird, wobei das Kontaktieren als kraftschlüssiges oder stoffschlüssiges Verbinden zwischen dem ersten Hohlkörper und dem weiteren Hohlkörper ausgeführt wird, und wobei die Pumpvorrichtung dazu geeignet ist, in den Körper eines Menschen oder eines Tieres eingebracht zu werden.

Pumpvorrichtungen mit Rotoren oder Impellern sind bekannt. Manche Pumpvorrichtungen weisen als Förderstrecke für ein zu förderndes Fluid ein Pumpengehäuse in Form eines Rohres auf. Darin befindet sich oftmals ein Impeller, der zum Beispiel von einem außerhalb der Förderstrecke gelegenen Motor über eine Antriebswelle angetrieben wird. Das Pumpengehäuse ist über ein oder mehrere Halteelemente an der Pumpvorrichtung befestigt. Diese Art der Halterung kann verschiedene Nachteile beinhalten. Zum einen wird ein zusätzlicher Arbeitsschritt zum Anbringen der Halterung benötigt. Das erhöht die Herstellungskosten und ist ressourcenineffizient. Weiterhin ist die Verbindung zwischen dem Pumpengehäuse und der Halterung herstellungsbedingt oder aufgrund der eingesetzten Verbindungsmittel, z.B. Schrauben oder Nieten, nicht ohne Spannung. Dies liegt daran, dass für die Halterungen und/oder Verbindungsmittel meist andere Materialien ausgewählt werden als für das Pumpengehäuse. Durch diese Spannungen verschlechtern sich die Verbindungen der Halterung mit dem Pumpengehäuse mit der Zeit. Darüber hinaus ist es vor allem für sehr kleine Pumpen äußerst wichtig platzsparend hergestellt zu werden. Dies gilt insbesondere für Pumpen, die in einen Körper implantiert werden sollen. Dies ist für Pumpen mit einer Vielzahl von Einzelteilen schwerer realisierbar als bei einer Pumpe mit einer kleineren Anzahl von Einzelteilen.

Allgemein liegt eine Aufgabe der vorliegenden Erfindung darin, die sich aus dem Stand der Technik ergebenden Nachteile zumindest teilweise zu überwinden.

Eine weitere Aufgabe besteht darin, eine Pumpvorrichtung bereitzustellen deren Materialien möglichst biokompatibel, leicht verarbeitbar, korrosionsbeständig und dauerhaft miteinander verbindbar sind.

Eine weitere Aufgabe besteht darin, eine Pumpvorrichtung bereitzustellen, die möglichst platzsparend ausgestaltet ist.

Weiterhin besteht eine Aufgabe darin, eine möglichst spannungsfreie Pumpvorrichtung bereitzustellen, insbesondere mit einem möglichst spannungsfreien Gehäuse bzw. Pumpengehäuse, und insbesondere einen möglichst spannungsfreien Übergang vom Pumpengehäuse zum restlichen Teil der Pumpvorrichtung bereitzustellen.

Es besteht zudem eine Aufgabe darin, eine Pumpvorrichtung bereitzustellen, die einen möglichst geringen Abrieb der beweglichen Teile und deren Halterungen bei Benutzung aufweist.

Darüber hinaus besteht eine Aufgabe darin, ein Pumpengehäuse für eine Pumpvorrichtung bereitzustellen, die einfach und platzsparend in andere Bauteile, z.B. ein Bauteilgehäuse der Pumpvorrichtung integrierbar ist.

Darüber hinaus besteht eine Aufgabe darin, ein Pumpengehäuse für eine Pumpvorrichtung bereitzustellen, das hermetisch dicht mit einem Bauteilgehäuse der Pumpvorrichtung verbunden werden kann.

Weiterhin besteht eine Aufgabe darin, ein Gehäuse oder Pumpengehäuse bereitzustellen, das möglichst frei von inneren und / oder äußeren Spannungen ist.

Weiterhin besteht eine Aufgabe darin, ein Verfahren bereitzustellen, um ein Pumpengehäuse möglichst kosten- und zeitsparend herstellen zu können.

Es ist weiterhin eine Aufgabe, ein Bauteilgehäuse bereitzustellen, das möglichst platzsparend ausgestaltet ist.

Eine weitere Aufgabe ist ein Gehäuse bereitzustellen, das hermetisch dicht mit anderen Bauteilen verbunden werden kann.

Ein erster Gegenstand der vorliegenden Erfindung ist eine Pumpvorrichtung (10), beinhaltend:
i. einen Impeller;
ii. ein Pumpengehäuse, das einen Innenbereich zumindest zu einem Teil umgibt, mit einem Einlass und einem Auslass,
wobei der Impeller im Innenbereich des Pumpengehäuses vorgesehen ist;
wobei das Pumpengehäuse einen Verbund aus einem ersten Hohlkörper und einem den ersten Hohlkörper auf der dem Innenbereich abgewandten Seite zumindest zum Teil umgebenden weiteren Hohlkörper beinhaltet;
wobei mindestens 60 % der Oberfläche der dem Innenraum abgewandten Seite des ersten Hohlkörpers mit dem weiteren Hohlkörper verbunden sind, bezogen auf die Oberfläche der dem Innenraum abgewandten Seite des ersten Hohlkörpers,
wobei das Pumpengehäuse mit einer weiteren Oberfläche des ersten Hohlkörpers dem Innenbereich zugewandt ist;
wobei die dem Innenbereich des Pumpengehäuses zugewandte weitere Oberfläche des ersten Hohlkörpers eine Härte von mindestens 330 HV aufweist,
wobei der weitere Hohlkörper einen Metallgehalt von mindestens 50 Gew.-%, bezogen auf die Gesamtmasse des weiteren Hohlkörpers beinhaltet.

Die erfindungsgemäße Pumpvorrichtung ist dazu geeignet in den Körper eines Menschen oder eines Tieres eingebracht zu werden. Die erfindungsgemäße Pumpvorrichtung ist ferner bevorzugt dazu ausgelegt, Körperflüssigkeiten wie Blut, Serum, Plasma, Interstitielle Flüssigkeit, Speichel oder Urin zu fördern. Insbesondere ist es bevorzugt, die erfindungsgemäße Pumpvorrichtung zum Fördern von Blut in den Blutkreislauf eines Menschen oder Tieres einzubringen. Das Einbringen der erfindungsgemäßen Pumpvorrichtung kann beispielsweise ein Implantieren in den Körper, ein Aufsetzen auf den Körper oder ein Verbinden mit dem Körper beinhalten.

Das Pumpengehäuse der erfindungsgemäßen Pumpvorrichtung kann jede Form aufweisen, die der Fachmann für den Einsatz in einer Pumpvorrichtung auswählen würde. Das Pumpengehäuse weist bevorzugt mindestens eine Wand des Pumpengehäuses, im Folgenden auch als Pumpengehäusewand bezeichnet, auf. Die mindestens eine Wand des Pumpengehäuses umgibt den Innenbereich des Pumpengehäuses. Das Pumpengehäuse weist mindestens zwei Enden auf, wobei mindestens ein Einlass an dem einen Ende und mindestens ein Auslass an dem anderen Ende angeordnet sind. Der Innenbereich des Pumpengehäuses ist, außer an dem Einlass und Auslass des Pumpengehäuses, von der Wand vollständig umgeben. Das Pumpengehäuse kann sich zum Teil über den Innenbereich des Pumpengehäuses hinaus erstrecken. Bevorzugt endet das Pumpengehäuse an dem Einlass bzw. Auslass.

Die dem Innenbereich abgewandte Seite des Pumpengehäuses wird als Außenseite des Pumpengehäuses bezeichnet. Das Pumpengehäuse weist bevorzugt eine längliche Form auf. Das Pumpengehäuse wird in seiner Form durch eine Längsausdehnung und mindestens einen Querschnitt definiert. Ein Querschnitt des Pumpengehäuses wird immer in einer Ebene bestimmt, die senkrecht zu der Pumpengehäusewand steht. Ist die Pumpengehäusewand in der Längsausdehnung gekrümmt, so wird ein Querschnitt senkrecht zur Tangente an einem Punkt auf der Pumpengehäusewand ermittelt. Als Längsausdehnung wird die Ausdehnung des Pumpengehäuses in Pumprichtung angesehen. Es gilt stets die kürzeste, gedachte Verbindung von Einlass und Auslass innerhalb des Pumpengehäuses. Die Pumpengehäusewand, auch als Wand bezeichnet, erstreckt sich in Richtung der Längsausdehnung des Pumpengehäuses. Die mindestens eine Wand kann eine oder mehrere Wandflächen aufweisen. Weist das Pumpengehäuse mehr als eine Wandfläche auf, sind diese über Ecken, an denen die Wandflächen zusammenlaufen, miteinander verbunden. Die Wand, sowie bevorzugt auch die Wandflächen, des Pumpengehäuses verlaufen bevorzugt parallel zur Längsausdehnung des Pumpengehäuses. Ein Teil der Pumpengehäusewand kann sich über den Innenbereich des Pumpengehäuses hinaus erstrecken. Bevorzugt erstreckt sich die Pumpengehäusewand über den gesamten Innenbereich des Pumpengehäuses. Ist das Pumpengehäuse röhrenförmig ausgestaltet, befinden sich der Einlass an dem ersten Ende und der Auslass an dem gegenüberliegenden Ende des Pumpengehäuses. An den Enden des Pumpengehäuses endet bevorzugt mindestens ein Teil der Pumpengehäusewand. Der Teil des Pumpengehäuses, der über den Innenbereich in die Umgebung hinaus ragt, wird als Pumpengehäusezunge bezeichnet. In einer bevorzugten Ausgestaltung der erfindungsgemäßen Pumpvorrichtung weist das Pumpengehäuse an dem ersten Ende, dem Einlass, eine erste Öffnung zu dem Innenbereich und an dem weiteren Ende, dem Auslass, eine weitere Öffnung zu dem Innenbereich auf. Über Einlass und Auslass ist das Pumpengehäuse mit seiner Umgebung fluidleitend verbunden. Die Öffnungen an den Enden des Pumpengehäuses ermöglichen ein Durchfließen eines Fluids durch den Innenbereiches des Pumpengehäuses. Das Fluid ist beispielsweise ein Gas, eine Flüssigkeit, wie Blut, oder einer Mischung hieraus. Bevorzugt dient die erste Öffnung als Zuleitung des zu fördernden Fluids in den Innenbereich des Pumpengehäuses und die weitere Öffnung als Ableitung des zu fördernden Fluids. Das Pumpengehäuse kann weitere Öffnungen aufweisen, beispielsweise in der Wand des Pumpengehäuses. Diese weiteren Öffnungen können zum zusätzlichen Zuleiten von Fluid oder auf der anderen Seite zum verzweigten Ableiten von Fluid dienen. Wird die erfindungsgemäße Pumpvorrichtung in einen Körper implantiert, um beispielsweise den Blutkreislauf zu unterstützen und damit das Herz zu entlasten, so wird die erfindungsgemäße Pumpvorrichtung über Leitungen an Blutgefäße des Körpers angeschlossen.

Das Pumpengehäuse beinhaltet mindestens einen Querschnitt auf, der bevorzugt ausgewählt ist aus der Gruppe bestehend aus kreisförmig, rechteckig oder vieleckig oder ellipsoid. Bevorzugt weist das Pumpengehäuse eine längliche Form mindestens in einem ersten Abschnitt auf. Weiterhin kann das Pumpengehäuse mindestens einen weiteren Abschnitt beinhalten, dessen Form von dem ersten Abschnitt des Pumpengehäuses abweicht.

Bevorzugt ist die Gesamtlänge des Pumpengehäuses 1,5- bis 10-mal, bevorzugt 2- bis 9-mal, oder bevorzugt 2,5- bis 8,5-mal länger als der Innendurchmesser des Pumpengehäuses. Die Länge des Pumpengehäuses wird bevorzugt entlang der Außenwand des Pumpengehäuses in Pumprichtung bestimmt. Das Pumpengehäuse weist bevorzugt eine Länge in einem Bereich von 1 mm bis 10 cm, oder bevorzugt in einem Bereich von 2 mm bis 8 cm, oder bevorzugt in einem Bereich von 5 mm bis 5 cm auf. Das Pumpengehäuse weist bevorzugt einen Innendurchmesser in einem Bereich von 0,1 bis 50 mm, oder bevorzugt in einem Bereich von 0,5 bis 30 mm, oder bevorzugt in einem Bereich von 1 bis 20 mm auf.

Die Wand, insbesondere die mindestens eine Wandfläche des Pumpengehäuses, ist bevorzugt glatt. Glatt bedeutet, dass die Wand des Pumpengehäuses eine Rauheit in einem Bereich von 0,025 bis 4 Ra, oder bevorzugt in einem Bereich von 0,05 bis 3 Ra, oder bevorzugt in einem Bereich von 0,07 bis 1 Ra aufweist.

Das Pumpengehäuse beinhaltet mindestens einen ersten Hohlkörper und mindestens einen weiteren Hohlkörper. Der erste und der weitere Hohlkörper unterscheiden sich bevorzugt durch ihre Zusammensetzung. Der mindestens eine erste Hohlkörper weist bevorzugt mindestens eine, besonders bevorzugt alle der folgenden Eigenschaften auf:
- möglichst hohe thermische Beständigkeit;
- möglichst hohe Druckbeständigkeit;
- möglichst hohe Härte;
- möglichst hohe Beständigkeit gegen Säuren und Basen;
- möglichst geringe Rauigkeit;
- möglichst spannungsfreie Verbindbarkeit mit einem Metall oder einem Metall-Keramik-Gemisch (Cermet);
- möglichst gute Versinterbarkeit mit einem Metall oder einem Metall-Keramik-Gemisch (Cermet);
- möglichst geringe elektrische Leitfähigkeit;
- möglichst geringe magnetische Permeabilität.

Der mindestens eine weitere Hohlkörper weist bevorzugt mindestens eine, bevorzugt alle der folgenden Eigenschaften auf:
- möglichst hohe thermische Beständigkeit;
- möglichst hohe Druckbeständigkeit;
- möglichst hohe Härte;
- möglichst hohe Beständigkeit gegen Säuren und Basen;
- möglichst geringe Rauigkeit;
- möglichst gute Verbindbarkeit mit einem Metall;
- möglichst gute Verschweißbarkeit mit einem Metall.
- möglichst hohe Wärmausdehnungskoeffizienten

Werden die beiden ersten und weiteren Hohlkörper bei der Herstellung des Pumpengehäuses erfindungsgemäß zusammengebracht, so kann ein Pumpengehäuse erhalten werden, das die für den mindestens einen ersten Hohlkörper und den mindestens einen weiteren Hohlkörper eine oder mehrere der aufgelisteten Eigenschaften vereinigt. Mindestens ein Teil des mindestens einen ersten Hohlkörpers ist mit mindestens einem Teil des weiteren Hohlkörpers verbunden. Die Verbindung kann eine unmittelbare Verbindung der beiden Hohlkörper sein oder eine mittelbare. Sind der mindestens eine erste Hohlkörper und der mindestens eine weitere Hohlkörper unmittelbar miteinander verbunden, so sind der erste Hohlkörper und der weitere Hohlkörper stoffschlüssig oder kraftschlüssig miteinander verbunden.

Eine stoffschlüssige Verbindung liegt bei dem unmittelbaren Verbinden dann vor, wenn die stofflichen Eigenschaften des ersten Hohlkörpers fließend in die stofflichen Eigenschaften des weiteren Hohlkörpers übergehen. Es liegt meistens keine scharfe Grenze zwischen den beiden Hohlkörpern vor. Vielmehr besteht meistens ein Übergangsbereich in dem sich die Eigenschaften der beiden Hohlkörper mischen. Dieser Übergangsbereich kann auch als Zwischenbereich bezeichnet werden. In diesem Zwischenbereich sind die Materialien des ersten Hohlkörpers zum Teil in die Materialien des weiteren Hohlkörpers eindiffundiert und bilden bevorzugt eine Gemengelage der Materialien. Bevorzugt gehen die Materialien der beiden Hohlkörper eine Verbindung auf atomarer oder molekularer Ebene ein. Es wirken Kräfte auf atomarer oder molekularer Ebene der Materialien der ersten und weiteren Hohlkörper. Eine stoffschlüssige Verbindung kann in der Regel nur durch Zerstörung der Materialien gelöst werden. Als Beispiel für solch eine stoffschlüssige Verbindung kann das Behandlung der verbundenen Hohlkörper bei einer Temperatur von bevorzugt über 500 °C, oder bevorzugt von über 1000 °C, oder bevorzugt von über 1500 °C genannt werden.

Eine weitere unmittelbare Verbindung der beiden Hohlkörper, bei der eine kraftschlüssige Verbindung zwischen den Hohlkörpern vorliegt ist ein Presspassen. Diese wird später näher erläutert.

Eine alternative stoffschlüssige Verbindung der beiden Hohlkörper ist beispielsweise ein Verkleben der beiden Hohlkörper an den Kontaktstellen. Auch das Verkleben wird später noch näher erläutert.

Der mindestens eine erste Hohlkörper beinhaltet bevorzugt zu mindestens 40 Gew.-%, bevorzugt zu mindestens 70 Gew.-%, oder bevorzugt zu mindestens 90 Gew.-%, bezogen auf die Gesamtmasse des ersten Hohlkörpers eine Keramik. Bevorzugt beinhaltet der mindestens eine erste Hohlkörper die Keramik in einem Bereich von 40 bis 100 Gew.-%, oder bevorzugt in einem Bereich von 70 bis 100 Gew.-%, oder bevorzugt in einem Bereich von 80 bis 100 Gew.-%, bezogen auf die Gesamtmasse des ersten Hohlkörpers. Weiterhin bevorzugt beinhaltet der mindestens eine erste Hohlkörper die Keramik zu 100 Gew.-%, bezogen auf die Gesamtmasse des ersten Hohlkörpers. Der mindestens eine erste Hohlkörper kann weitere Materialien beinhalten. Die weiteren Materialien können ausgewählt sein aus der Gruppe bestehend aus Wasser, einem Additiv, einem Cermet oder einer Mischung von mindestens zwei hiervon

Die Keramik kann jede Keramik sein, die der Fachmann für die erfindungsgemäße Pumpvorrichtung auswählen würde. Die Keramik ist bevorzugt ausgewählt aus der Gruppe bestehend aus einer Oxidkeramik, einer Silikatkeramik, einer Nichtoxid-Keramik oder einer Mischung aus mindestens zwei davon.

Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Beryllium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid (Al₂O₃), Magnesiumoxid (MgO), Zirkoniumoxid (ZrO₂), Yttriumoxid (Y₂O₃), Aluminiumtitanat (Al₂TiO₅), einer Piezokeramik wie Blei-Zirkonat (PbZrO₃), Blei-Titanat (PbTiO₃) sowie Blei-Zirkonat-Titanat (PZT) oder einer Mischung von mindestens zwei hiervon. Das Halbmetall des Halbmetalloxids ist bevorzugt ausgewählt aus der Gruppe bestehend aus Bor, Silicium, Arsen, Tellur oder einer Mischung von mindestens zwei davon.

Die Silikatkeramik ist bevorzugt ausgewählt aus der Gruppe bestehend aus einem Steatit (Mg₃[Si₄O₁₀(OH)₂]), Cordierit (Mg, Fe²⁺)₂(Al₂Si)[Al₂Si₄O₁₈]), Mullit (Al₂Al₂₊₂ₓSi₂₋₂ₓO₁₀₋ₓ mit x = Sauerstoffleerstellen pro Elementarzelle), Feldspat (Ba,Ca,Na,K,NH₄)(AL,B,Si)₄O₈) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid (B₄C), Titancarbid (TiC), Wolframcarbid, Zementit (Fe3C). Das Nitrid kann ausgewählt sein aus der Gruppe bestehend aus Siliciumnitrid (Si₃N₄), Aluminiumnitrid (AIN), Titannitrid (TiN), Siliciumaluminiumoxinitrid (SIALON) oder einer Mischung aus mindestens zwei davon.

Im Rahmen der Erfindung wird als "Cermet" ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix verstanden. Zur Herstellung eines Cermets kann beispielsweise ein Gemisch aus mindestens einem keramischen Pulver und mindestens einem metallischen Pulver verwendet werden, welches beispielsweise mit mindestens mit einem Bindemittel und gegebenenfalls mindestens einem Lösungsmittel versetzt werden kann. Eine Auswahl für die keramischen Bestandteile und die metallischen Bestandteile des Cermets können sich aus denen zusammensetzen, die für den ersten Hohlkörper angegeben wurden.

Der mindestens eine weitere Hohlkörper oder dessen mindestens eine weitere Hohlkörpervorläufer beinhaltet das Additiv bevorzugt in einer Menge in einem Bereich von 0,1 bis 5 Gew.-%, oder bevorzugt in einem Bereich von 0,2 bis 2 Gew.-%, oder bevorzugt in einem Bereich von 0,3 bis 1 Gew.-%, jeweils bezogen auf das Gesamtgewicht des mindestens einen weiteren Hohlkörpers bzw. des Hohlkörpervorläufers.

Der mindestens eine erste Hohlkörper und der mindestens eine weitere Hohlkörper können auf unterschiedliche Weise innerhalb des Pumpengehäuses angeordnet sein. In einer bevorzugten Ausgestaltung des Pumpengehäuses weist mindestens eine Oberfläche des mindestens einen weiteren Hohlkörpers zur Außenseite des Pumpengehäuses hin und mindestens eine Oberfläche des mindestens einen ersten Hohlkörpers zum Innenbereich. Diese wird auch als die dem Innenbereich abgewandte Seite bezeichnet. Der mindestens eine erste Hohlkörper oder der mindestens eine weitere Hohlkörper kann mindestens in einem Teil des Pumpengehäuses die gesamte Wandstärke in einem Querschnitt des Pumpengehäuses an einem Punkt bilden. Die Wand des Pumpengehäuses wird dann an diesen Stellen des Querschnitts nur von einem einzigen Hohlkörper gebildet, also dem ersten Hohlkörper oder dem zweiten Hohlkörper. Alternativ kann die Wand des Pumpengehäuses in einem Querschnitt durch an einem Punkt am Pumpengehäuse mindestens einen ersten Hohlkörpers und mindestens einen weiteren Hohlkörpers aufweisen. Bevorzugt weist der erste Hohlkörper zur Seite des Innenbereiches des Pumpengehäuses, während der weitere Hohlkörper bevorzugt zur Außenseite des Pumpengehäuses weist. Dies kann zum Beispiel durch Ineinanderstellen von zwei Rohren erreicht werden, wobei das erste Rohr für den ersten Hohlkörper steht und das zweite Rohr für den weiteren Hohlkörper steht.

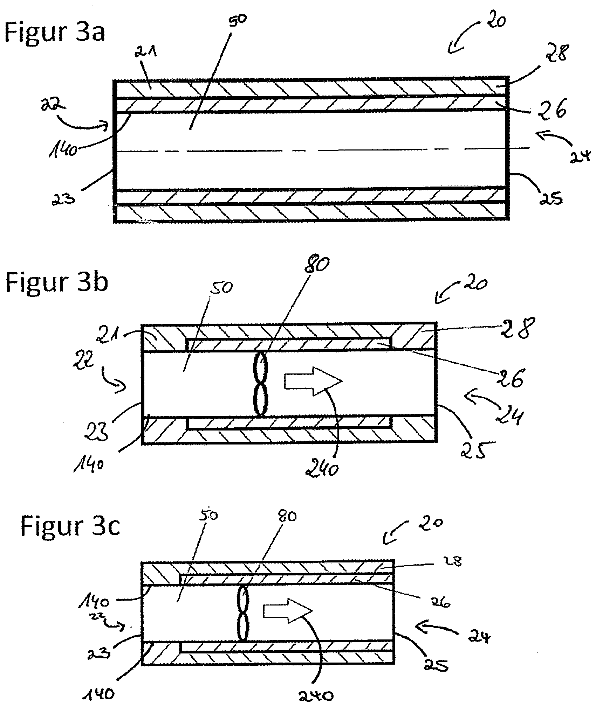

Jeder Übergang von einem Hohlkörper zu einem anderen Hohlkörper kann in Bezug auf einen Querschnitt des Pumpengehäuses rechtwinklig oder in einem von 90 ° verschiedenen Winkel angeordnet sein. Ferner kann jeder Übergang auch unregelmäßig ausgebildet sein, d.h. im Querschnitt kann keine gedachte grade Linie auf dem Übergang angelegt werden. Weiterhin kann jeder Übergang von einem Hohlkörper zu einem anderen Hohlkörper alternativ oder zusätzlich zu dem vorstehend beschriebenen in Bezug auf einen Längsschnitt durch eine Wand des Pumpengehäuses rechtwinklig oder in einem von 90 ° verschiedenen Winkel angeordnet sein. Ferner kann jeder Übergang auch unregelmäßig ausgebildet sein, d.h. im Längsschnitt kann keine gedachte grade Linie auf dem Übergang angelegt werden. Ferner sind Kombinationen der vorstehend genannten Konfigurationen eines Übergangs im Querschnitt und im Längsschnitt bevorzugt. Beispiele hierzu sind in Figur 3a bis 3e gezeigt.

Bevorzugt weist das Pumpengehäuse entlang seiner Längsausdehnung verschiedene Abschnitte auf, in denen das Pumpengehäuse entweder nur den ersten Hohlkörper oder nur den weiteren Hohlkörper, oder beide Hohlkörper beinhaltet. Diese Abschnitte können sich über die gesamte Länge des Pumpengehäuses abwechseln.

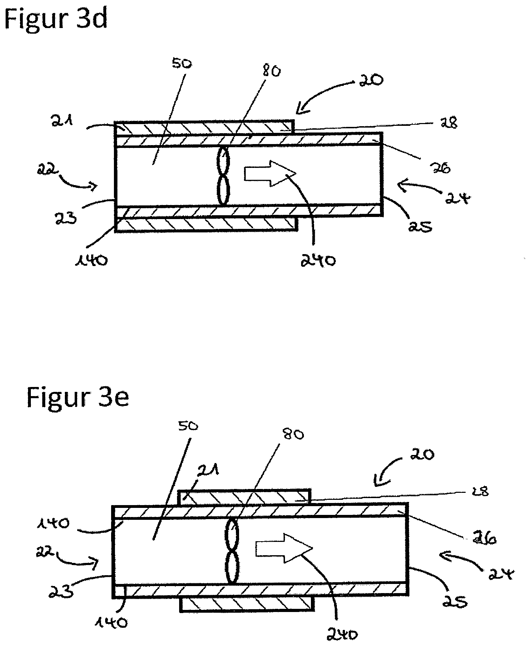

In einer bevorzugten Ausgestaltung des Pumpengehäuses der erfindungsgemäßen Pumpvorrichtung erstrecken sich sowohl der erste Hohlkörper als auch der weitere Hohlkörper über die gesamte Länge des Pumpengehäuses. Dies ist exemplarisch in Figur 3a gezeigt. In einer anderen Ausgestaltung des Pumpengehäuses der erfindungsgemäßen Pumpvorrichtung erstreckt sich der weitere Hohlkörper über die gesamte Länge des Pumpengehäuses, der erste Hohlkörper nur über einen Teil der Länge des Pumpengehäuses. Dies ist exemplarisch in Figur 3b und 3c gezeigt. Weiterhin erstrecken sich in einer anderen Ausgestaltung des Pumpengehäuses der erfindungsgemäßen Pumpvorrichtung der erste Hohlkörper über die gesamte Länge des Pumpengehäuses und der weitere Hohlkörper nur über einen Teil der Länge des Pumpengehäuses. Dies ist exemplarisch in Figur 3d und 3e gezeigt. In einer wiederum anderen Ausgestaltung des Pumpengehäuses der erfindungsgemäßen Pumpvorrichtung weist das Pumpengehäuse an dem Einlass lediglich den ersten Hohlkörper oder den weiteren Hohlkörper auf. Dies ist exemplarisch in Figur 3b, 3c, und 3e gezeigt. Eine weitere bevorzugt Ausgestaltung des Pumpengehäuses der erfindungsgemäßen Pumpvorrichtung weist an dem Auslass des Pumpengehäuses lediglich den ersten Hohlkörper oder den weiteren Hohlkörper auf. Dies ist exemplarisch in Figur 3b, 3d und 3e gezeigt.

Weiterhin ist es in einer Ausgestaltung des Pumpengehäuses der erfindungsgemäßen Pumpvorrichtung bevorzugt, wenn ein Teil der Oberfläche des weiteren Hohlkörpers zum Innenbereich weist. Dies ist exemplarisch in Figur 3b und 3c gezeigt. In einer weiteren bevorzugten Ausgestaltung des Pumpengehäuses der erfindungsgemäßen Pumpvorrichtung weist ausschließlich die weitere Oberfläche des ersten Hohlkörpers zum Innenbereich hin. Dies ist exemplarisch in Figur 3a, 3d und 3e gezeigt.

Die erfindungsgemäße Pumpvorrichtung beinhaltet zudem einen Rotor in Form des Impellers. Der Impeller kann jede Form aufweisen, die der Fachmann hierfür auswählen würde.

Der Impeller weist bevorzugt einen Durchmesser in einem Bereich von 1 mm bis 10 cm, bevorzugt in einem Bereich von 3 mm bis 5 cm, oder bevorzugt in einem Bereich von 5 mm bis 3 cm auf. Der Impeller weist bevorzugt eine Dicke in einem Bereich von 0,1 bis 50 mm, bevorzugt in einem Bereich von 0,5 bis 20 mm, oder bevorzugt in einem Bereich von 1 bis 15 mm auf. Der Durchmesser des Impellers ist bevorzugt kleiner als der Durchmesser des Pumpengehäuses in der Ebene des Impellers. Der Durchmesser des Impellers ist bevorzugt in einem Bereich von 1 bis 10 %, oder bevorzugt in einem Bereich von 1,5 bis 8 %, oder bevorzugt in einem Bereich von 2 bis 7 %, bezogen auf den Durchmesser des Pumpengehäuses in der Ebene des Impellers, kleiner als der Durchmesser des Pumpengehäuses.

Der Impeller weist bevorzugt mindestens zwei Rotorblätter auf, bevorzugt mindestens drei Rotorblätter, oder bevorzugt mindestens fünf Rotorblätter. Besonders bevorzugt weist der Impeller eine Anzahl von Rotorblättern in einem Bereich von 2 bis 20, bevorzugt in einem Bereich von 5 bis 15, oder bevorzugt in einem Bereich von 8 bis 13 auf. Der Impeller weist bevorzugt eine zentrale Drehachse auf, um die der Impeller gedreht werden kann. Die Drehachse wird auch als Rotationsachse bezeichnet. Die mindestens zwei Rotorblätter sind bevorzugt symmetrisch um die Drehachse des Impellers angeordnet. Der Impeller ist bevorzugt im Innerbereich des Pumpengehäuses angeordnet, wobei die Rotationsachse des Impellers parallel zur Längsausdehnung der Wand des Rohres vorgesehen ist.

Der Impeller kann aus jedem Material hergestellt sein, das der Fachmann für einen Einsatz in der erfindungsgemäßen Pumpvorrichtung auswählen würde. Bevorzugt weist der Impeller mindestens zwei Bereiche auf: Einen ersten Bereich im Zentrum des Impellers um die Rotationsachse herum. Dieser erste Bereich wird auch Kernbereich genannt. Einen zweiten Bereich, auch Rotorbereich genannt. Dieser zweite Bereich weist mindestens zwei Rotorblätter auf, die zur Förderung des zu fördernden Fluids geeignet sind.

Der Impeller beinhaltet mindestens ein Element, wobei das Element hartmagnetische Eigenschaften aufweist. Eine hartmagnetische Eigenschaft bedeutet, dass ein Material eine dauerhafte Magnetisierung in Folge von Aussetzen dieses Materials in einem Magnetfeld erhält. Die Stärke eines magnetisierenden Feldes wird in Abhängigkeit von der Zusammensetzung des Elementes gewählt. Die dazu notwendigen Überlegungen und Berechnungen sind dem Fachmann geläufig. Bevorzugt wird beim Magnetisieren die Induktion des Impellers gesättigt. Nach Abfall des Magnetfeldes besteht die Magnetisierung des hartmagnetischen Materials weiter. Materialien mit hartmagnetischen Eigenschaften können als Dauermagnete eingesetzt werden. Das mindestens eine Elemente ist bevorzugt so an dem Impeller angeordnet, dass es den Impeller bewegt, wenn es alternierend von zwei voneinander unabhängigen elektrischen bzw. magnetischen Feldern angezogen oder abgestoßen wird. Der Impeller beinhaltet bevorzugt mindestens zwei Elemente mit hartmagnetischen Eigenschaften. Weiterhin kann durch mindestens ein optionales Element der Impeller in seiner radialen aber auch axialen Ausrichtung gesteuert werden. Bevorzugt werden die Elemente mit hartmagnetischen Eigenschaften dazu genutzt, den Impeller ohne weitere Hilfsmittel, wie Lagerungen oder sonstige Fixierungen in dem Pumpengehäuse möglichst kontaktlos im Pumpengehäuse zu lagern. Dies ermöglicht einen besonders reibungsarmen und besonders verschleißarmen Betrieb.

Das mindestens eine Element kann beispielsweise durch mindestens ein Rotorblatt verwirklicht werden, das ein hartmagnetisches Material beinhaltet. Alternativ kann an mindestens einem Rotorblatt ein hartmagnetisches Element angeordnet sein. Bevorzugt ist das hartmagnetische Element im Kern des Impellers vorgesehen. Das mindestens eine hartmagnetische Element beinhaltet bevorzugt mindestens ein magnetisierbares Material, wie Eisen, Kobalt, Nickel, Chromdioxid oder eine Mischung von mindestens zwei hiervon. Das mindestens eine Element kann beispielsweise in Form einer Beschichtung aus hartmagnetischem Material auf mindestens einem Rotorblatt oder im Innern des Impellers angeordnet sein. Bevorzugt beinhalten mindestens 50 %, oder bevorzugt mindestens 70 %, oder bevorzugt 100 % der Rotorblätter ein hartmagnetisches Material. Bevorzugt beinhaltet das Element zu mindestens 10 Gew.-%, oder bevorzugt zu mindestens 20 Gew.-%, oder bevorzugt zu mindestens 30 Gew.-%, bezogen auf die Gesamtmasse des Elementes ein hartmagnetisches Metall. Weiterhin bevorzugt beinhaltet das Element eine Kobalt-Chrom-Legierung oder eine Platin-Kobalt-Legierung, insbesondere eine Platin-Kobalt-Legierung (PtCo23) mit einem Anteil an Kobalt von 23 Gew.-% bezogen auf die Gesamtmasse der Legierung, in einem Bereich von 10 bis 100 Gew.-%, oder bevorzugt in einem Bereich von 20 bis 100 Gew.-%, oder bevorzugt in einem Bereich von 30 bis 100 Gew.-%, bezogen auf die Gesamtmasse des Elementes.

Der Impeller kann in seinem Kern, dem Bereich um die Drehachse herum, ein anderes Material aufweisen als in oder an den Rotorblättern. Alternativ kann der Impeller ein einheitliches Material im Kern und den Rotorblättern beinhalten. Das Material der Rotorblätter kann flexibel oder unflexibel sein. Bevorzugt ist das Material des Kerns des Impellers oder der Rotorblätter des Impellers jeweils ausgewählt aus der Gruppe bestehend aus einem Polymer, einem Metall, einer Keramik oder einer Kombination oder Mischung aus mindestens zwei hiervon.

Das Polymer kann ausgewählt sein aus der Gruppe bestehend aus einem Chitosan, einem Fibrin, einem Collagen, einem Caprolacton, einem Lactid, einem Glycolid, einem Dioxanon, einem Polyurethan, einem Polyimid, einem Polyamid, einem Polyester, einem Polymethylmethacrylat, einem Polyacrylat, einem Teflon, einem Copolymer aus mindestens zwei hieraus oder einer Mischung aus mindestens zwei hiervon.

Das Metall kann ausgewählt sein aus der Gruppe bestehend aus Eisen (Fe), Edelstahl, Platin (Pt), Iridium (Ir), Niob (Nb), Molybdän (Mo), Wolfram (W), Titan (Ti), Kobalt (Co), Chrom (Cr), eine Kobalt-Chrom-Legierung, Tantal (Ta), Vanadium (V) und Zirkonium (Zr) oder einer Mischung aus mindestens zwei hiervon, wobei insbesondere bevorzugt sind Titan, Niob, Molybdän, Kobalt, Chrom, Tantal, Zirkonium, Vanadium und deren Legierungen.

Die Keramik kann ausgewählt sein aus der Gruppe bestehend aus Aluminiumoxid (Al₂O₃), Zirkoniumdioxid (ZrO₂), Hydroxilapatit, Tricalciumphosphat, Glaskeramik, Aluminiumoxid verstärktes Zirkoniumoxid (ZTA), Zirkoniumoxid enthaltendes Aluminiumoxid (ZTA - Zirconia Toughened Aluminum - Al₂O₃/ZrO₂), Yttrium enthaltendes Zirkoniumoxid (Y-TZP), Aluminiumnitrid (AIN), Magnesiumoxid (MgO), Piezokeramik, Barium(Zr, Ti)oxid, Barium(Ce, Ti)oxid und Natrium-Kalium-Niobat oder einer Mischung aus mindestens zwei hiervon.

Weiterhin bevorzugt kann der Impeller an seiner Außenseite, insbesondere an der Außenfläche der Rotorblätter, mit einem biokompatiblen Material beschichtet sein. Geeignete biokompatible Materialien werden im folgenden beschrieben.

Der Impeller ist in dem Pumpengehäuse bevorzugt in dem ersten Hohlkörper des Pumpengehäuses angeordnet und ist also von dem ersten Hohlkörper umgeben. Der Impeller ist bevorzugt mit seiner Rotationsachse parallel zur Längsausdehnung der Wand angeordnet. Weiterhin kann der Impeller durch ein Magnetfeld in dem Pumpengehäuse ausgerichtet werden. Der Impeller in dem Innenbereich des Pumpengehäuses wird bevorzugt von magnetischen Feldern von elektrischen Spulen an der Außenseite des Pumpengehäuses ausgerichtet. Die Spulen beinhalten bevorzugt ein elektrisch leitendes Material. Bevorzugt ist das elektrisch leitende Material der Spulen ausgewählt aus der Gruppe bestehend aus Eisen (Fe), Kupfer (Cu), Gold (Au), Silber (Ag), Platin (Pt), Palladium (Pd), Titan (Ti), Chrom (Cr), Kobalt (Co), Wolfram (W) oder einer Mischung aus mindestens zwei hiervon. Weiterhin bevorzugt beinhaltet das elektrisch leitende Material Kupfer (Cu). Die erfindungsgemäße Pumpvorrichtung beinhaltet bevorzugt mindestens zwei Spulen, bevorzugt mindestens drei Spulen, oder bevorzugt mindestens vier Spulen. Die Spulen sind bevorzugt an der Außenseite des Pumpengehäuses angeordnet, wobei die Spulen und der Impeller bevorzugt in einer Ebene liegen. Sie sind dann an der Außenseite des Pumpengehäuses um den Impeller herum angeordnet.

In einer bevorzugten Ausgestaltung der erfindungsgemäßen Pumpvorrichtung sind der erste Hohlkörper und der weitere Hohlkörper durch einen Kraftschluss verbunden.

In einer bevorzugten Ausgestaltung der erfindungsgemäßen Pumpvorrichtung bildet der erste Hohlkörper oder der weitere Hohlkörper einen zylindrischen Hohlkörper. Bevorzugt bilden der erste und der weitere Hohlkörper jeweils einen zylindrischen Hohlkörper.

In einer bevorzugten Ausgestaltung der erfindungsgemäßen Pumpvorrichtung beinhaltet das Pumpengehäuse ein Rohr. Bevorzugt ist das Rohr gerade. Alternativ kann das Rohr mindestens eine Biegung aufweisen. Das Rohr ist bevorzugt bis auf einen Einlass wie einen Auslass geschlossen. Das bedeutet, dass das Rohr außer den beiden Öffnungen am Einlass und Auslass keine weiteren Öffnungen aufweist. Die Dimensionen, Materialien und Ausgestaltungen entsprechen bevorzugt ansonsten denen des zuvor beschriebenen Pumpengehäuses.

In einer bevorzugten Ausgestaltung der erfindungsgemäßen Pumpvorrichtung ist das Material des ersten Hohlkörpers eine Keramik, die bevorzugt ausgewählt aus der Gruppe bestehend aus Aluminiumoxid (Al₂O₃), Zirkoniumdioxid (ZrO₂), Aluminiumoxid verstärktes Zirkoniumoxid (ZTA), Zirkoniumoxid verstärktes Aluminiumoxid [(ZTA - Zirconia Toughened Aluminum - Al₂O₃/ZrO₂)], Yttrium verstärktes Zirkoniumoxid (Y-TZP), Aluminiumnitrid (AIN), Magnesiumoxid (MgO), Piezokeramik, Barium(Zr, Ti)oxid, Barium(Ce, Ti)oxid, Titannitrid (TiN), Siliziumdioxid (SiO₂) und Natrium-Kalium-Niobat oder einer Mischung aus mindestens zwei hiervon.

Der mindestens eine weitere Hohlkörper des Pumpengehäuses beinhaltet einen Metallgehalt in einem Bereich von 50 bis 90 Gew.-%, bevorzugt in einem Bereich von 55 bis 85 Gew.-%, oder bevorzugt in einem Bereich von 60 bis 80 Gew.-%, bezogen auf die Gesamtmasse des weiteren Hohlkörpers.

In einer bevorzugten Ausgestaltung der erfindungsgemäßen Pumpvorrichtung ist das Metall des weiteren Hohlkörpers ausgewählt aus der Gruppe bestehend Platin (Pt), Eisen (Fe), Edelstahl, insbesondere AISI 304 oder AISI 316 L, Iridium (Ir), Niob (Nb) eine Niob-Legierung, Molybdän (Mo), Wolfram (W), Titan (Ti), eine Titan-Legierung, Kobalt (Co), Chrom (Cr), eine Kobalt-Chrom-Legierung, Tantal (Ta) und Zirkonium (Zr) oder einer Mischung aus mindestens zwei hiervon. Bevorzugt ist das Metall ausgewählt aus der Gruppe bestehen aus Titan, Niob, Molybdän, Kobalt, Chrom, Tantal und deren Legierungen oder einer Mischung aus mindestens zwei hiervon.

Weiterhin kann der mindestens eine weitere Hohlkörper weitere Materialien aufweisen. Das weitere Material des weiteren Hohlkörpers kann, wie bereits für den ersten Hohlkörper beschrieben, ausgewählt sein aus der Gruppe bestehend aus einem Additiv, einer Keramik, einem Cermet oder einer Mischung hiervon. Der mindestens eine weitere Hohlkörper beinhaltet das weitere Material bevorzugt in einem Bereich von 10 bis 650 Gew.-%, oder bevorzugt in einem Bereich von 11 bis 545 Gew.-%, oder bevorzugt in einem Bereich von 13 bis 40 Gew.-%.

Die Keramik des weiteren Hohlkörpers kann jede Keramik sein, die der Fachmann für eine Pumpvorrichtung auswählen würde. Die Keramik ist bevorzugt ausgewählt aus der Gruppe bestehend aus einer Oxidkeramik, einer Silikatkeramik, einer Nichtoxid-Keramik oder einer Mischung aus mindestens zwei davon. Die Keramik des mindestens einen weiteren Hohlkörpers kann aus der gleichen Gruppe ausgewählt sein wie die für den ersten Hohlkörper aufgeführten Keramiken. Bevorzugt weist der mindestens eine weitere Hohlkörper die gleiche Keramik auf wie der mindestens eine weitere Hohlkörper. Der weitere Hohlkörper beinhaltet die Keramik bevorzugt in einem Bereich von 1 bis 50 Gew.-%, oder bevorzugt in einem Bereich von 5 bis 45 Gew.-%, oder bevorzugt in einem Bereich von 10 bis 40 Gew.-%, bezogen auf die Gesamtmasse des weiteren Hohlkörpers.

Weiterhin kann der mindestens eine weitere Hohlkörper ein Additiv beinhalten, wie es für den ersten Hohlkörper beschrieben wurde. Das Additiv beinhaltet bevorzugt ein Material ausgewählt aus der Gruppe bestehend aus Wasser, einem Dispergiermittel, einem Bindemittel oder einer Mischung von mindestens zwei hiervon. Alle weiteren Eigenschaften und Mengenangaben zu den Additiven können den Angaben zu dem ersten Hohlkörper entnommen werden. Die Summe aller Bestandteile des weiteren Hohlkörpers ergibt stets 100 Gew.-%.

Eine Auswahl für die keramischen Bestandteile und die metallischen Bestandteile des Cermets können sich aus denen zusammensetzen, die für den mindestens einen weiteren Hohlkörper angegeben wurden.

Das Pumpengehäuse beinhaltet mindestens einen ersten Hohlkörper und mindestens einen weiteren Hohlkörper. Das Pumpengehäuse kann mehrere erste Hohlkörper und mehrere weitere Hohlkörper aufweisen. Bevorzugt weist das Pumpengehäuse je einen ersten und einen weiteren Hohlkörper auf. Der mindestens eine erste und der mindestens eine weitere Hohlkörper können gleich groß sein oder alternativ unterschiedliche Größen aufweisen. Der mindestens eine erste Hohlkörper und der mindestens eine weitere Hohlkörper erstrecken sich größtenteils zusammen bevorzugt über die gesamte Dicke der Pumpengehäusewand. Die Dicke der ersten und weiteren Hohlkörper sind bevorzugt zueinander identisch und entsprechen, je nach Anordnung der Hohlkörper zueinander entweder jeweils einzeln oder zusammen der Wanddicke wie für die Pumpengehäusewand oben angegeben. Der mindestens eine erste Hohlkörper weist bevorzugt eine Breite, bezogen auf die Längsausdehnung des Pumpengehäuses, in einem Bereich von 1 bis 100 mm, bevorzugt in einem Bereich von 2 bis 70 mm, oder bevorzugt in einem Bereich von 3 bis 50 mm auf. Der mindestens eine weitere Hohlkörper weist bevorzugt eine Breite in den gleichen Bereichen wie der erste Hohlkörper auf. Weist das Pumpengehäuse mehr als einen ersten und/oder mehr als einen weiteren Hohlkörper auf, so sind die mehreren ersten und/oder mehreren weiteren Hohlkörper bevorzugt gleich breit. Alternativ können sich unterschiedliche breite erste und unterschiedlich breite weitere Hohlkörper abwechseln.

Weiterhin kann der mindestens eine erste Hohlkörper ein Metall beinhalten. Das Metall kann jedes Metall sein, das der Fachmann für die Fertigung des Pumpengehäuses auswählen würde.

In einer bevorzugten Ausgestaltung der erfindungsgemäßen Pumpvorrichtung beinhaltet der mindestens eine erste Hohlkörper weniger als 10 Gew.-%, bevorzugt von weniger als 5 Gew.-%, oder bevorzugt von weniger als 3 Gew.-%, bezogen auf die Gesamtmasse des ersten Hohlkörpers, an Metall. Die Summe aller Bestandteile des ersten Hohlkörpers ergibt stets 100 Gew.-%.

Das Metall des ersten Hohlkörpers ist bevorzugt ausgewählt aus der Gruppe bestehend aus Platin (Pt), Eisen (Fe), Edelstahl (AISI 304, AISI 316 L), Iridium (Ir), Niob (Nb), Molybdän (Mo), Wolfram (W), Titan (Ti), Kobalt (Co), Chrom (Cr), eine Kobalt-Chrom-Legierung, Tantal (Ta), und Zirkonium (Zr) oder einer Mischung aus mindestens zwei hiervon. Bevorzugt ist das Metall ausgewählt aus der Gruppe bestehen aus Titan, Niob, Molybdän, Kobalt, Chrom, Tantal und deren Legierungen oder einer Mischung aus mindestens zwei hiervon. Bevorzugt weist der erste Hohlkörper das gleiche Metall auf wie der weitere Hohlkörper.

In einer bevorzugten Ausgestaltung der Pumpvorrichtung ist die Pumpvorrichtung zumindest zu einem Teil von einem Bauteilgehäuse umgeben, wobei mindestens ein Teil des mindestens einen weiteren Hohlkörpers der Pumpvorrichtung mit dem Bauteilgehäuse verbunden ist. Die Verbindung des Bauteilgehäuses mit mindestens einem Teil des weiteren Hohlkörpers des Pumpengehäuses führt bevorzugt zu einem abgeschlossenen Raum zwischen dem Bauteilgehäuse und dem Pumpengehäuse. Bevorzugt ist das Innere des Bauteilgehäuses der Pumpvorrichtung hermetisch gegen die Umwelt abgeschlossen. Die hier erfindungsgemäß vorgeschlagene medizinisch implantierbare Pumpvorrichtung kann insbesondere in einen Körper eines menschlichen oder tierischen Benutzers, insbesondere eines Patienten, eingesetzt werden. Eine implantierte Pumpvorrichtung ist in der Regel einer Flüssigkeit eines Körpergewebes des Körpers ausgesetzt. Somit ist es in der Regel von Bedeutung, dass weder Körperflüssigkeit in die medizinisch implantierbare Vorrichtung eindringt, noch das Flüssigkeiten aus der medizinisch implantierbaren Vorrichtung austreten. Um dieses sicherzustellen, sollte das Bauteilgehäuse der medizinisch implantierbaren Vorrichtung, und somit auch das Bauteilgehäuse sowie das Pumpengehäuse der erfindungsgemäßen Pumpvorrichtung, eine möglichst vollständige Undurchlässigkeit aufweisen, insbesondere gegenüber Körperflüssigkeiten.

Die erfindungsgemäße Pumpvorrichtung, insbesondere die Verbindungen von Bauteilgehäuse mit Pumpengehäuse sind bevorzugt hermetisch dicht, So ist der Innenraum der Pumpvorrichtung hermetisch dicht gegenüber dem Außenraum abgedichtet. Im Rahmen der Erfindung bedeutet der Begriff "hermetisch dicht", dass bei einem bestimmungsgemäßen Gebrauch innerhalb eines üblichen Zeitraums von 5 Jahren keine Feuchtigkeit und/oder Gase die hermetisch dichte Verbindung durchdringen können. Eine physikalische Größe, zum Bestimmen der Dichtheit einer Verbindung oder eines Bauteils ist die Leckrate. Dichtheiten können durch Lecktests bestimmt werden. Lecktests werden mit Heliumlecktestern und/oder Massenspektrometern durchgeführt werden und sind im Standard Mil-STD-883G Method 1014 spezifiziert. Die maximal zulässige Helium-Leckrate wird dabei abhängig vom internen Volumen der zu prüfenden Vorrichtung festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, beträgt die maximal zulässige Helium-Leckrate für die erfindungsgemäßen Pumpengehäuse 10⁻⁷ atm*cm³/sec oder weniger. Das bedeutet, dass die zu prüfende Vorrichtung (beispielsweise das Bauteilgehäuse und/oder die erfindungsgemäße Pumpvorrichtung oder das Bauteilgehäuse mit dem verbundenen Pumpengehäuse) eine Helium-Leckrate von weniger als 1 x 10⁻⁷ atm*cm³/sec oder weniger aufweist. In einer besonders vorteilhaften Ausführung beträgt die Helium-Leckrate weniger als 1 x 10⁻⁸ atm*cm³/sec, insbesondere weniger als 1 x 10⁻⁹ atm*cm³/sec. Zum Zweck der Standardisierung können die genannten Helium-Leckraten auch in die äquivalente Standard-Luft-Leckrate konvertiert werden. Die Definition für die äquivalente Standard-Luft-Leckrate (Equivalent Standard Air Leak Rate) und die Umrechnung sind im Standard ISO 3530 angegeben.

Die erfindungsgemäße Pumpvorrichtung weist bevorzugt neben dem Impeller, dem Pumpengehäuse mit einem ersten Hohlkörper und einem weiteren Hohlkörper bevorzugt ein Bauteilgehäuse auf, in dem sich weitere Bauteile der Pumpvorrichtung befinden können. Die weiteren Bauteile der Pumpvorrichtung sind bevorzugt ausgewählt aus der Gruppe bestehend aus einer Batterie, einer Spule, einer Steuereinheit, einer Gefäßverbindungseinheit oder einer Kombination aus mindestens zwei hieraus.

In einer bevorzugten Ausgestaltung der erfindungsgemäßen Pumpvorrichtung weist das Pumpengehäuse ein Volumen in einem Bereich von 0,1 cm³ bis 10 cm³, bevorzugt in einem Bereich von 0,2 bis 9 cm³, oder bevorzugt in einem Bereich von 0,5 bis 5 cm³ auf. Bevorzugt sind die Dimensionen wie Länge, Durchmesser und Wanddicke des Pumpengehäuses wie bereits oben angegeben. Das Volumen des Pumpengehäuses wird bevorzugt durch seinen Innenraum und die Wandstärke des Pumpengehäuses definiert. Die Wand des Pumpengehäuses weist bevorzugt eine Stärke bzw. Dicke in einem Bereich von 0,1 bis 5 mm, oder bevorzugt in einem Bereich von 0,3 bis 4 mm, oder bevorzugt in einem Bereich von 0,4 bis 3 mm auf. Das Volumen des Innenbereiches berechnet sich entsprechend aus der Länge und dem Innendurchmesser des Pumpengehäuses. Die bevorzugten Bereiche für das Innenvolumen ergeben sich ebenfalls dementsprechend. Im Folgenden wird in diesem Zusammenhang entweder von Wandstärke oder Wanddicke gesprochen. An der Innenfläche des Pumpengehäuses können in mindestens einem der ersten oder der weiteren Hohlkörper die Wanddicken variieren. Eine Erhöhung der Wanddicke an mindestens einem Punkt des Pumpengehäuses kann dazu dienen, den Impeller mindestens in eine Richtung an seiner Position im Pumpengehäuse zu halten.

In einer bevorzugten Ausgestaltung der erfindungsgemäßen Pumpvorrichtung beinhaltet das Bauteilgehäuse Titan zu mindestens 30 Gew.-%, bevorzugt mindestens 50 Gew.-%, oder bevorzugt mindestens 80 Gew.-%, jeweils bezogen auf die Gesamtmasse des Bauteilgehäuses. Weiterhin bevorzugt beinhaltet das Bauteilgehäuse Titan zu mindestens 99 Gew.-%, bezogen auf die Gesamtmasse des Bauteilgehäuses. Weiterhin kann das Bauteilgehäuse bevorzugt mindestens ein anderes Metall beinhalten. Das andere Metall kann aus der gleichen Gruppe ausgewählt sein, wie das Metall des weiteren Hohlkörpers. Das andere Metall ist bevorzugt ausgewählt aus der Gruppe bestehend aus Fe, Al, V, Sn, Co, Cr, CoCr, Nb, Edelstahl, Mb, TiNb oder einer Mischung von mindestens zwei hiervon. Das Bauteilgehäuse kann das weitere Metall bevorzugt in einem Bereich von 1 bis 70 Gew.-%, oder bevorzugt in einem Bereich von 5 bis 50 Gew.-%, oder bevorzugt in einem Bereich von 10 bis 20 Gew.-% beinhalten. Die Summe aller Bestandteile des Bauteilgehäuses ergibt stets 100 Gew.-%. Geeignete Titanqualitäten sind in ASTM B265-05, Jahr 2011 angegeben, zum Beispiel Grade 1 bis 6.

In einer bevorzugten Ausgestaltung der erfindungsgemäßen Pumpvorrichtung weist die Wand des Pumpengehäuses eine magnetische Permeabilität von weniger als 2 µ, bevorzugt weniger als 1,9 µ, oder bevorzugt weniger als 1,8 µ auf. Die magnetische Permeabilität wird gemäß der Norm ASTM 773:2009, Variante 01 bestimmt. Die Messdauer beträgt 40 Sekunden.

In der erfindungsgemäßen Pumpvorrichtung weist die weitere Oberfläche des ersten Hohlkörpers, die dem Innenbereich des Pumpengehäuses zugewandt ist, eine Härte nach Vickers von mindestens 330 HV, bevorzugt mindestens 350 HV, oder bevorzugt mindestens 370 HV auf. Bevorzugt weist der gesamte mindestens eine erste Hohlkörper eine Härte in den angegeben Bereichen auf. Mindestens die Außenoberfläche des mindestens einen weiteren Hohlkörpers weist ebenfalls eine Härte nach Vickers von mindestens 330 HV, bevorzugt mindestens 350 HV, oder bevorzugt mindestens 370 HV auf. Oftmals ist die Härte nicht höher als 2000 HV, oder bevorzugt nicht höher als 1500 HV. Bevorzugt liegt die Härte mindestens der Oberfläche des mindestens einen ersten Hohlkörpers in einem Bereich von 330 bis 2000 HV, oder bevorzugt in einem Bereich von 350 bis 1800 HV. Weiterhin bevorzugt weist mindestens die weitere Oberfläche des mindestens einen ersten Hohlkörpers eine Härte auf, die mindestens so groß ist wie die Härte der Rotoroberflächen des Impellers. Bevorzugt weist mindestens die Oberfläche des mindestens einen ersten Hohlkörpers eine Härte auf, die um mindestens 20 HV, oder bevorzugt um mindestens 30 HV, oder bevorzugt um mindestens 40 HV höher liegt als die Härte nach Vickers der Rotoroberflächen des Impellers. Als Oberfläche des mindestens einen einen Hohlkörpers, des mindestens einen weiteren Hohlkörpers sowie des Impellers wird die oberflächennahe Materieschicht in einem Bereich von 0,01 bis 2,5 mm, bevorzugt in einem Bereich von 0,05 bis 1,5 mm, oder bevorzugt in einem Bereich von 0,1 bis 1 mm, jeweils senkrecht zur Oberfläche verstanden.

In einer bevorzugten Ausgestaltung der erfindungsgemäßen Pumpvorrichtung sind zumindest die Außenflächen des Bauteilgehäuses und die dem Innenbereich des Pumpengehäuses zugewandte Oberfläche biokompatibel. Dies ist insbesondere bevorzugt, wenn die Pumpvorrichtung für die Implantation in einen lebenden Körper, wie beispielsweise den eines Menschen oder Tieres. Die Biokompatibilität wird ermittelt und beurteilt gemäß der Norm ISO 10993-4:2002.

In der Regel kommen die dem Innenbereich des Pumpengehäuses zugewandten Oberflächen und die Außenflächen des Bauteilgehäuses nach Implantieren der Pumpvorrichtung in einen lebenden Körper mit dessen Körperflüssigkeit in Kontakt. Die Biokompatibilität der mit Körperflüssigkeit in Berührung kommenden Oberflächen trägt dazu bei, dass der Körper beim Kontakt mit diesen Flächen keinen Schaden nimmt.

Ein weiterer Gegenstand der vorliegenden Erfindung ist ein Verfahren zur Herstellung eines Pumpengehäuses für eine Pumpvorrichtung beinhaltend die Schritte:
a. Bereitstellen eines ersten Hohlkörpers;
b. Bereitstellen eines weiteren Hohlkörpers;
   wobei mindestens der erste Hohlkörper als zylindrischer Hohlkörper ausgebildet ist;
c. Kontaktieren des ersten Hohlkörpers mit dem weiteren Hohlkörper,
wobei ein Verbund gebildet wird,
wobei das Kontaktieren als kraftschlüssiges oder stoffschlüssiges Verbinden zwischen dem ersten Hohlkörper und dem weiteren Hohlkörper ausgeführt wird, und wobei die Pumpvorrichtung dazu geeignet ist, in den Körper eines Menschen oder eines Tieres eingebracht zu werden.

Das Bereitstellen des ersten Hohlkörpers in Schritt a. und des weiteren Hohlkörpers in Schritt b. kann auf jede beliebige Art und Weise erfolgen, die der Fachmann für diesen Zweck auswählen würde.

Das Kontaktieren in Schritt c. kann jedes Kontaktieren sein, das der Fachmann für eine kraftschlüssige oder stoffschlüssige Verbindung zwischen dem ersten und dem weiteren Hohlkörper verwenden würde.

In einer bevorzugten Ausgestaltung des Verfahrens zur Herstellung eines erfindungsgemäßen Pumpengehäuses ist das Kontaktieren in Schritt c. ausgewählt aus der Gruppe bestehend aus einem Kleben, einem Pressen, einem Sintern oder einer Kombination aus mindestens zwei davon. Bevorzugt ist das Kontaktieren ausgewählt aus der Gruppe bestehend aus einem Pressen mit Heißkontaktieren, einem Sintern oder einem Kleben oder einer Kombination aus mindestens zwei hiervon. Erfindungsgemäß wird in dem Schritt c. ein Verbund aus dem ersten Hohlkörper und dem weiteren Hohlkörper hergestellt. Dieser Verbund der beiden Hohlkörper bildet ein stabiles Pumpengehäuse. Der Verbund zwischen den beiden Hohlkörpern ist bevorzugt so stark, dass der Versuch der Trennung der beiden Hohlkörper zu einer Zerstörung des Pumpengehäuses führen würde.

Das Heißkontaktieren wird bevorzugt mittels eines Erhitzens eines der beiden Hohlkörper vorgenommen. Bevorzugt wird der weitere Hohlkörper auf eine Temperatur erhitzt, die zwar unterhalb der Schmelztemperatur des weiteren Hohlkörpers liegt, jedoch eine Ausdehnung des Hohlkörpers mit sich bringt. Bevorzugt weist der weitere Hohlkörper ebenfalls eine zylindrische Form auf. Weiterhin bevorzugt weist der weitere Hohlkörper einen Innendurchmesser auf, der identisch ist mit dem Außendurchmesser des ersten Hohlkörpers. Bevorzugt wird der Hohlkörper auf eine erhöhte Temperatur gebracht, sodass sich sein Innendurchmesser sich etwas erhöht und der weitere Hohlkörper über den ersten Hohlkörper geschoben werden kann. Bevorzugt wird der weitere Hohlkörper auf eine Temperatur in einem Bereich von 200 bis 400 °C, oder bevorzugt in einem Bereich von 220 bis 350 °C, oder bevorzugt in einem Bereich von 240 bis 300 °C erhitzt. Beim Abkühlen des weiteren Hohlkörpers verringert sich dessen Durchmesser und es wird eine kraftschlüssige Verbindung zwischen dem ersten und dem weiteren Hohlkörper gebildet. Eine kraftschlüssige Verbindung zwischen zwei Gegenständen wie dem ersten und dem weiteren Hohlkörper kann eine genauso feste Verbindung erzeugen wie eine stoffschlüssige Verbindung, wie sie beispielsweise beim Kleben oder Sintern entsteht.

Das Kontaktieren durch Kleben, kann mit jeden Kleber durchgeführt werden, den der Fachmann hierfür auswählen würde. Die beiden Hohlkörper weisen dabei beide bevorzugt eine zylindrische Form auf. Der Innendurchmesser des weiteren Hohlkörpers ist bevorzugt etwas größer als der Außendurchmesser des ersten Hohlkörpers. Der weitere Hohlkörper muss dabei keine geschlossene Längsausdehnung aufweisen. Es kann sich beispielsweise bei dem weiteren Hohlkörper um einen geschlitzten länglichen, hohlen Körper handeln.

Der Kleber kann ausgewählt sein aus der Gruppe bestehend aus Polyurethan, Polyacrylate, Polyester, Polyvinylalkohole, Polysulfone oder eine Mischung aus mindestens zwei hiervon. Ein Lösungsmittel für die Polymere ist bevorzugt ausgewählt aus der Gruppe bestehend aus Dimethylsulfoxid (DMSO), Ethylenglykol, N-Methyl-2-pyrrolidon (NMP), Ammoniak, Wasser, einem Alkohol, wie beispielsweise Ethanol, Isopropanol oder Hexanol, oder einer Mischung aus mindestens zwei hiervon. Weiterhin kann beispielsweise ein Vernetzer eingesetzt werden. Der Vernetzer kann beispielsweise ein Silan sein.

In einer weiteren Ausführungsform des erfindungsgemäßen Verfahrens kann der erste Hohlkörper bereits als gebrannte Keramik vorliegen. Bevorzugt liegt der erste Hohlkörper als ein Rohr mit einer Länge von 2 cm mit einem Innendurchmesser von 9 mm. Auf die Keramik wird dann bevorzugt mindestens eine Schicht eines weiteren Materials, aus dem der weitere Hohlkörper gebildet wird, per Siebdruck an mindestens einer Stelle des ersten Hohlkörpers aufgebracht. Der Vorgang des Siebdruckens kann so lange wiederholt werden, bis eine ausreichende Schicht, beispielsweise in einem Bereich von 0,2 bis 5 mm, oder bevorzugt in einem Bereich von 0,5 bis 4,5 mm, oder bevorzugt in einem Bereich von 0,6 bis 4,3 mm an weiterem Material für den weiteren Hohlkörper auf dem Ende des ersten Hohlkörpers aufgebracht ist. Alternativ können beide Hohlkörper nacheinander per Siebdruck hergestellt werden. Das Kontaktieren beim Sintern wird bevorzugt durch ein Kontaktieren von zwei Vorläufern der Hohlkörper vorgenommen. Diese können zusammen auch als Pumpengehäusevorläufer bezeichnet werden. Bevorzugt wird hierzu zunächst ein zylindrischer Vorläufer eines ersten Hohlkörpers, bevorzugt als ungebrannte Masse eines Keramikmaterials wie zuvor für den ersten Hohlkörper beschrieben bereitgestellt. Auf die äußere Oberfläche wird anschließend ein ungebranntes Material ausgewählt aus den Bestandteilen des weiteren Hohlkörpers wie für die Pumpvorrichtung zuvor beschrieben aufgebracht. Das ungebrannte Material liegt bevorzugt als Paste oder Pulver vor. Die beiden sich wenigstens zum Teil umhüllenden ersten und weiteren Hohlkörper werden zusammen einem Sinterprozess unterzogen. Der Sinterprozess findet bevorzugt bei einer Temperatur in einem Bereich von 500 bis 2500 °C, oder bevorzugt in einem Bereich von 700 bis 2000 °C, oder bevorzugt in einem Bereich von 1000 bis 1700 °C statt. Die Behandlung des Hohlkörpervorläufers wird über einen Zeitraum in einem Bereich von 0,1 bis 100 h, bevorzugt in einem Bereich von 1 bis 50 h, oder bevorzugt in einem Bereich von 2 bis 20 h vorgenommen.

Weiterhin kann der mindestens eine erste Hohlkörpervorläufer und der mindestens eine weitere Hohlkörpervorläufer ein Additiv aufweisen. Das Additiv kann ausgewählt sein aus der Gruppe bestehend aus Wasser, einem Dispergiermittel, einem Bindemittel oder einer Mischung von mindestens zwei hiervon.

Das Dispergiermittel beinhaltet bevorzugt mindestens eine organische Substanz. Die organische Substanz weist bevorzugt mindestens eine funktionale Gruppe auf. Die funktionale Gruppe kann ein hydrophobe oder eine hydrophile funktionale Gruppe sein. Die funktionale Gruppe kann ausgewählt sein aus der Gruppe bestehend aus einer Ammonium-Gruppe, einer Carboxylat-Gruppe, einer Sulfat-Gruppe, einer Sulfonat-Gruppe, einer Alkohol-Gruppe, einer Mehrfachalkohol-Gruppe, einer Ether-Gruppe oder einer Mischung aus mindestens zwei hiervon. Das Dispergiermittel weist funktionale Gruppen bevorzugt in einem Bereich von 1 bis 100, oder bevorzugt in einem Bereich von 2 bis 50, oder bevorzugt in einem Bereich von 2 bis 30 auf. Bevorzugte Dispergiermittel sind unter den Handelsnamen DISPERBYK® 60 von Byk-Chemie GmbH, DOLAPIX CE 64 von Zschimmer & Schwarz GmbH & Co KG erhältlich.

Das Bindemittel ist bevorzugt ausgewählt aus der Gruppe bestehend aus einer Methylcellulose, einem thermoplastischen Polymer, einem duroplastischen Polymer und einem Wachs oder einer Mischung von mindestens zwei hiervon.

Die Methylcellulose ist bevorzugt ausgewählt aus der Gruppe bestehend aus Hydroxypropylmethylcellulose (HPMC), Hydroxyethylmethylcellulose (HEMC), Ethylmethylcellulose (EMC) oder einer Mischung daraus. Die Methylcellulose beinhaltet bevorzugt Hydroxypropylmethylcellulose (HPMC) Weiterhin bevorzugt beinhaltet die Methylcellulose Hydroxypropylmethylcellulose in einem Bereich von 80 bis 100 Gew.-%, oder bevorzugt in einem Bereich von 90 bis 100 Gew.-%, oder bevorzugt in einem Bereich von 95 bis 100 Gew.-%, bezogen auf die Gesamtmasse an Methylcellulose. Bevorzugt weist die Methylcellulose einen Anteil an -OCH₃ Gruppen in einem Bereich von 20 bis 40 Gew.-%, oder bevorzugt in einem Bereich von 23 bis 37 Gew.-%, oder bevorzugt in einem Bereich von 25 bis 35 Gew.-%, bezogen auf die Gesamtmasse an Methylcellulose auf. Weiterhin bevorzugt weist die Methylcellulose einen Anteil an -OC₃H₆OH Gruppen in einem Bereich von 1 bis 12 Gew.-%, oder bevorzugt in einem Bereich von 3 bis 9 Gew.-%, oder bevorzugt in einem Bereich von 4 bis 8 Gew.-%, bezogen auf die Gesamtmasse an Methylcellulose auf.

Das thermoplastische Polymer kann ausgewählt sein aus der Gruppe bestehend aus AcrylnitrilButadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) und Polyvinylchlorid (PVC) oder einer Mischung von mindestens zwei davon. Das duroplastische Polymer kann ausgewählt sein aus der Gruppe bestehend aus einem Aminoplasten, einem Epoxidharz, einem Phenolharz, einem Polyester-Harz oder einer Mischung aus mindestens zwei davon. Wachse sind Kohlenwasserstoffverbindungen, die oberhalb 40 °C ohne Zersetzung schmelzen. Hierunter können sich auch Polyester, Paraffine, Polyethylene oder Copolymere aus mindestens zwei daraus befinden.

Das Material zur Bildung des ersten Hohlkörpers oder des weiteren Hohlkörpers beinhaltet bevorzugt mindestens eines der vorgenannten Additive bevorzugt in einem Bereich von 0,1 bis 10 Gew.-%, oder bevorzugt in einem Bereich von 0,2 bis 8 Gew.-%, oder bevorzugt in einem Bereich von 0,5 bis 5 Gew.-%, bezogen auf die Gesamtmasse des ersten Materials.

In einer Ausführungsform des Prozesses kann der erste Hohlkörper bereits als gebrannte Keramik vorliegen. Bevorzugt liegt der erste Hohlkörper als ein Rohr mit einer Länge von 2 cm mit einem Innendurchmesser von 9 mm. Auf die Keramik wird dann bevorzugt mindestens eine Schicht eines weiteren Materials, aus dem der weitere Hohlkörper gebildet wird, per Siebdruck an mindestens einer Stelle des ersten Hohlkörpers aufgebracht. Der Vorgang des Siebdruckens kann so lange wiederholt werden, bis eine ausreichende Schicht, beispielsweise in einem Bereich von 0,2 bis 5 mm, oder bevorzugt in einem Bereich von 0,5 bis 4,5 mm, oder bevorzugt in einem Bereich von 0,6 bis 4,3 mm an weiterem Material für den weiteren Hohlkörper auf dem Ende des ersten Hohlkörpers aufgebracht ist. Alternativ können beide Hohlkörper nacheinander per Siebdruck hergestellt werden. Das Kontaktieren beim Sintern wird bevorzugt durch ein Kontaktieren von zwei Vorläufern der Hohlkörper vorgenommen. Diese können zusammen auch als Pumpengehäusevorläufer bezeichnet werden. Bevorzugt wird hierzu zunächst ein zylindrischer Vorläufer eines ersten Hohlkörpers, bevorzugt als ungebrannte Masse eines Keramikmaterials wie zuvor für den ersten Hohlkörper beschrieben bereitgestellt. Auf die äußere Oberfläche wird anschließend ein ungebranntes Material ausgewählt aus den Bestandteilen des weiteren Hohlkörpers wie für die Pumpvorrichtung zuvor beschrieben aufgebracht. Das ungebrannte Material liegt bevorzugt als Paste oder Pulver vor. Die beiden sich wenigstens zum Teil umhüllenden ersten und weiteren Hohlkörper werden zusammen einem Sinterprozess unterzogen. Der Sinterprozess findet bevorzugt bei einer Temperatur in einem Bereich von 500 bis 2500 °C, oder bevorzugt in einem Bereich von 700 bis 2000 °C, oder bevorzugt in einem Bereich von 1000 bis 1700 °C statt. Die Behandlung des Hohlkörpervorläufers wird über einen Zeitraum in einem Bereich von 0,1 bis 100 h, bevorzugt in einem Bereich von 1 bis 50 h, oder bevorzugt in einem Bereich von 2 bis 20 h vorgenommen. Weiterhin kann der mindestens eine erste Hohlkörpervorläufer und der mindestens eine weitere Hohlkörpervorläufer ein Additiv aufweisen. Das Additiv kann ausgewählt sein aus der Gruppe bestehend aus Wasser, einem Dispergiermittel, einem Bindemittel oder einer Mischung von mindestens zwei hiervon.

Weiterhin kann ein Zerspanen mit jedem anderen Formgebungsverfahren, insbesondere dem Kontaktieren in Schritt c. kombiniert werden. Beim Zerspanen wird ein massiver Körper durch Einsatz von Zerspanungshilfsmitteln, wie einem Bohrer oder einem Stempel strukturiert. Bei dem Strukturieren wird ein Teil des Materials abgetragen. Hierdurch können massive Körper beispielsweise zu Hohlkörpern geformt werden. Beispielsweise kann durch Zerspanen in den Pumpengehäusevorläufer ein Hohlraum geformt werden, wenn der Pumpengehäusevorläufer massiv ausgestaltet ist. Das Zerspanen kann jedoch auch ein Bearbeitungsschritt nach der Herstellung eines Pumpengehäuses oder Gehäuses sein. Zusätzlich zum Zerspanen kann im Anschluss an die Herstellung des Pumpengehäuses auch ein Polieren stattfinden.

Bei der Sinterbehandlung des Pumpengehäusevorläufers bei erhöhter Temperatur entweicht bevorzugt mindestens ein Teil des Bindemittels. Es sind verschiedene Temperaturprofile beim Behandeln in Schritt c. des Pumpengehäusevorläufers möglich. Das Behandeln des Pumpengehäusevorläufers kann beispielsweise in einer oxidativen Atmosphäre, einer reduktiven Atmosphäre oder unter einer Schutzatmosphäre erfolgen. Eine oxidative Atmosphäre kann beispielsweise Sauerstoff enthalten, wie Luft oder ein Sauerstoff/Luft Gemisch. Eine reduktive Atmosphäre kann beispielsweise Wasserstoff enthalten. Eine Schutzatmosphäre beinhaltet bevorzugt weder Sauerstoff noch Wasserstoff. Beispiele für Schutzatmosphären sind Stickstoff, Helium, Argon, Krypton oder deren Gemische. Die Wahl der Atmosphäre kann abhängig von den zu behandelnden Materialien sein. Dem Fachmann ist die geeignete Wahl der Atmosphäre für die erwähnten Materialien bekannt. Es können auch bevorzugt nacheinander Kombinationen von unterschiedlichen Atmosphären für verschiedene Zeiträume gewählt werden.

Das Behandeln des Pumpengehäusevorläufers kann entweder in einem Schritt erfolgen oder bevorzugt in mehr als einem Schritt. Bevorzugt wird der Pumpengehäusevorläufer in einem ersten Teilschritt auf eine Temperatur in einem Bereich von 301 bis 600 °C, oder bevorzugt in einem Bereich von 350 bis 550 °C, oder bevorzugt in einem Bereich von 400 bis 500 °C behandelt. Dieser erste Teilschritt kann über einen Zeitraum in einem Bereich von 0,1 bis 100 h, bevorzugt in einem Bereich von 1 bis 50 h, oder bevorzugt in einem Bereich von 2 bis 20 h erfolgen. Dieser Teilschritt kann entweder durch Einbringen des Pumpengehäusevorläufers aus Schritt c. in eine vorgeheizte Atmosphäre erfolgen oder durch langsames schrittweises oder stetig erhöhtes Erhitzen des Pumpengehäusevorläufers. Bevorzugt wird das Behandeln in dem ersten Teilschritt des Pumpengehäusevorläufers in einem Schritt auf eine Temperatur in einem Bereich von 301 bis 600 °C vorgenommen.

In einem zweiten Teilschritt des Behandelns, der sich bevorzugt an den ersten Teilschritt anschließt, wird der Pumpengehäusevorläufer bevorzugt auf eine Temperatur in einem Bereich von 800 bis 2500 °C, oder bevorzugt in einem Bereich von 1000 bis 2000 °C, oder bevorzugt in einem Bereich von 1100 bis 1800 °C erhitzt. Auch dieser Teilschritt kann entweder durch Einbringen des Pumpengehäusevorläufers aus dem ersten Teilschritt in eine vorgeheizte Atmosphäre erfolgen oder durch langsames schrittweises oder stetig erhöhtes Erhitzen des Pumpengehäusevorläufers. Bevorzugt wird das Behandeln in dem zweiten Teilschritt des Pumpengehäusevorläufers in einem Schritt auf eine Temperatur in einem Bereich von 800 bis 2500 °C vorgenommen. Die Behandlung des Pumpengehäusevorläufers in dem zweiten Teilschritt wird über einen Zeitraum in einem Bereich von 1 bis 180 min, bevorzugt in einem Bereich von 10 bis 120 min, oder bevorzugt in einem Bereich von 20 bis 100 min vorgenommen.

Die Form des Pumpengehäuses nach dem Herstellungsprozess ist bevorzugt kontinuierlich. Das bedeutet, dass das Pumpengehäuse neben dem Auslass und dem Einlass keine weiteren Öffnungen oder Auslässe, oder sonstige Aussparungen aufweist. Bevorzugt weist das Pumpengehäuse eine geradlinige Außenfläche auf. An der Innenfläche des Pumpengehäuses können in mindestens einem der ersten oder der weiteren Hohlkörper die Wandstärken variieren. Eine Erhöhung der Wandstärke an mindestens einem Punkt des Pumpengehäuses kann dazu dienen, den Impeller mindestens in eine Richtung an seiner Position im Pumpengehäuse zu halten. Die Verdickung der Wandstärke kann dabei entweder bereits während des Herstellprozesses stattfinden oder im Anschluss daran. Ergänzend oder alternativ kann das Pumpengehäuse Einschnürungen aufweisen.

Eine erfindungsgemäße Pumpvorrichtung ist erhältlich durch Einsetzen eines Impellers in ein Pumpengehäuse, Anordnen von Elektromagneten mit Spulen um das Pumpengehäuse, Herstellen eines Stromkreises unter Einbeziehen einer Steuervorrichtung und einer Stromquelle, z.B. einer Batterie. Bevorzugt wird die erfindungsgemäße Pumpvorrichtung von einem Bauteilgehäuse umgeben und der mindestens eine weitere Hohlkörper des Pumpengehäuses mit dem Bauteilgehäuse stoffschlüssig verbunden. Dies kann zum Beispiel durch eine Lötverbindung entlang der Berührungspunkt von Pumpengehäuses und Bauteilgehäuse durchgeführt werden.

Ein weiterer Gegenstand der vorliegenden Erfindung ist ein Pumpengehäuse für eine Pumpvorrichtung erhältlich nach dem zuvor beschriebenen erfindungsgemäßen Verfahren.

Ein weiterer Gegenstand der vorliegenden Erfindung ist ein Gehäuse, das einen Innenbereich zumindest zu einem Teil umgibt, mit einem ersten Ende und einem zweiten Ende,
wobei das Gehäuse einen Verbund aus einem ersten Hohlköper und einem den ersten Hohlkörper auf der dem Innenbereich abgewandten Seite zumindest zum Teil umgebenden weiteren Hohlkörper beinhaltet;
wobei mindestens 60 % der Oberfläche der dem Innenraum abgewandten Seite des ersten Hohlkörpers mit dem weiteren Hohlkörper verbunden sind, bezogen auf die Oberfläche der dem Innenraum abgewandten Seite des ersten Hohlkörpers;
wobei das Gehäuse mit einer weiteren Oberfläche des ersten Hohlkörpers dem Innenbereich zugewandt ist;
wobei die dem Innenbereich des Gehäuses zugewandte weitere Oberfläche des ersten Hohlkörpers eine Härte von mindestens 330 HV aufweist,
wobei der weitere Hohlkörper einen Metallgehalt von mindestens 50 Gew.-%, bezogen auf die Gesamtmasse des weiteren Hohlkörpers, beinhaltet, und wobei das Gehäuse dazu geeignet ist, in den Körper eines Menschen oder eines Tieres eingebracht zu werden.

Das Gehäuse entspricht in seiner Form, seiner Zusammensetzung und seiner sonstigen Ausgestaltung dem Pumpengehäuse, das zuvor im Zusammenhang mit der Pumpvorrichtung beschrieben wurde.

In einer bevorzugten Ausgestaltung des Gehäuses ist in dem Bauteilgehäuse zumindest in einem Teil des Bauteilgehäuses ein verschiebbares Element vorgesehen ist. Weitere bevorzugte Ausführungsformen entsprechen den zuvor beschriebenen Ausführungsformen der Pumpvorrichtung

Das verschiebbare Element kann ausgewählt sein aus der Gruppe bestehend aus einer Kugel, einem Zylinder, einer Luftblase oder einer Kombination aus mindestens zwei hiervon. Das verschiebbare Element weist bevorzugt eine Form auf, die dem Durchmesser des Pumpengehäuses entspricht. Das Material des verschiebbaren Elements kann jedes sein, das der Fachmann hierfür verwenden würde. Bevorzugt beinhaltet das verschiebbare Element ein Metall, ein Polymer, eine Keramik oder eine Mischung hieraus. Das Metall oder das Polymer kann ausgewählt sein aus einem Metall, einem Polymer oder einer Keramik wie sie für den ersten Hohlkörper für das Pumpengehäuse beschrieben wurde. Das verschiebbare Element kann dazu dienen beispielsweise durch eine Änderung des Fluidstroms in dem Bauteilgehäuse in seiner Position in dem Bauteilgehäuse verschoben zu werden. Die Änderung der Position kann beispielsweise durch Anlegen eines Magnetfeldes über dem Bauteilgehäuse detektiert werden.

Ein weiterer Gegenstand der vorliegenden Erfindung ist eine Pumpvorrichtung beinhaltend mindestens ein zuvor beschriebenes Gehäuse oder ein Pumpengehäuse erhältlich nach einem zuvor beschriebenen Verfahren.

### Messmethoden

1. Bestimmung der Härte nach Vickers (HV):
   Die Prüfkräfte und Materialien wurden gemäß der Norm DIN EN ISO 6507-März 2006 bestimmt. Es wurden folgende Prüfkräfte und Einwirkdauern verwendet: 1kg, 15 Sekunden. Die Prüftemperatur betrug 23 °C ± 1 °C
2. Bestimmung der magnetischen Permeabilität: Die magnetische Permeabilität wurde gemäß der Norm ASTM 773, 2009, Variante 01 bestimmt
3. Bestimmung der Biokompatibilität:
   Die Biokompatibilität wird gemäß der Norm ISO 10993-4:2002, bestimmt.
4. Bestimmung der Hermetischen Verbindung:
   Lecktests werden mit Heliumlecktestern und/oder Massenspektrometern durchgeführt werden. Ein Standardmessverfahren ist im Standard Mil-STD-883G Method 1014 spezifiziert. Die maximal zulässige Helium-Leckrate wird dabei abhängig vom internen Volumen der zu prüfenden Vorrichtung festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, beträgt die maximal zulässige Helium-Leckrate für die erfindungsgemäßen Pumpengehäuse 10⁻⁷ atm*cm³/sec oder weniger. Das bedeutet, dass die zu prüfende Vorrichtung (beispielsweise das Bauteilgehäuse und/oder die Pumpvorrichtung oder das Bauteilgehäuse mit dem verbundenen Pumpengehäuse) eine Helium-Leckrate von weniger als 1 x 10⁻⁷ atm*cm³/sec oder weniger aufweist. Für Vergleichszwecke können die genannten Helium-Leckraten auch in die äquivalente Standard-Luft-Leckrate konvertiert werden. Die Definition für die äquivalente Standard-Luft-Leckrate (Equivalent Standard Air Leak Rate) und die Umrechnung sind im Standard ISO 3530 angegeben.
5. Bestimmung der Rauheit: DIN EN ISO 4288. Weitere Parameterangaben: Maximaler Tastspitzenradius = 2 µm; Messstrecke = 1,25 mm; Grenzwellenlänge = 250 µm.

### Beispiele

### Beispiel 1 für erstes Material:

Das erste Material enthält 20 Teile Aluminiumoxid (Al₂O₃) erhältlich bei der Firma CeramTech GmbH, mit einer Korngröße von D₉₀ = 2 µm und einen Teil des Bindemittels METAWAX P-50 erhältlich bei der Firma Zschimmer & Schwarz GmbH & Co.KG.

### Beispiel 2 für weiteres Material:

Das weitere Material enthält eine Mischung aus 57 Gew.-% eines Platinpulvers der Firma Heraeus Precious Metals GmbH & Co.KG mit einer Korngröße D₅₀ = 50 µm, und 38 Gew.-% Aluminiumoxid (Al₂O₃) der Firma CeramTech GmbH mit einer Korngröße von D₉₀ = 2 µm sowie 5 Gew.-% eines Bindemittels METAWAX P-50 erhältlich bei der Firma Zschimmer & Schwarz GmbH & Co.KG.

### Beispiel 3 für ersten Hohlkörper:

Der erste Hohlkörper enthält zu 100 Gew.-% Aluminiumoxid (Al₂O₃) der Firma CeramTech GmbH.

### Beispiel 4 für weiteren Hohlkörper:

Der weitere Hohlkörper enthält zu 60 Gew.-% Platin und zu 40 Gew.-% Aluminiumoxid (Al₂O₃) der Firma CeramTech GmbH.

Das erste Material aus Beispiel 1 wird zunächst zu einem ersten Hohlkörpervorläufer verarbeitet. Das weitere Material aus Beispiel 2 wird zunächst zu einem weiteren Hohlkörpervorläufer verarbeitet, der den ersten Hohlköpervorläufer mindestens zu einem Teil umgibt. Auf diese Weise wird ein erfindungsgemäßer Pumpengehäusevorläufer erhalten. Durch Erhitzen des Pumpengehäusevorläufers in einem Bereich von 1400 bis 1800 °C wird ein erfindungsgemäßes Pumpengehäuse erhalten, das den ersten Hohlkörper mit der Zusammensetzung aus Beispiel 3 enthält und einen weiteren Hohlkörper mit einer Zusammensetzung, die der des Beispiel 4 entspricht.

Alternativ können die beiden Hohlkörpervorläufer separat voneinander gesintert werden und anschließend kraft oder stoffschlüssig miteinander verbunden werden.

Im Folgenden wird in
- Figur 1: eine schematische Darstellung einer erfindungsgemäßen Pumpvorrichtung;
- Figur 2: ein Schema eines Verfahrens zur Herstellung eines erfindungsgemäßen Pumpengehäuses;
- Figur 3a-e: verschiedene schematische Darstellungen eines erfindungsgemäßen Pumpengehäuses mit einem ersten und einem weiteren Hohlkörper direkt benachbart zueinander angeordnet;
gezeigt.

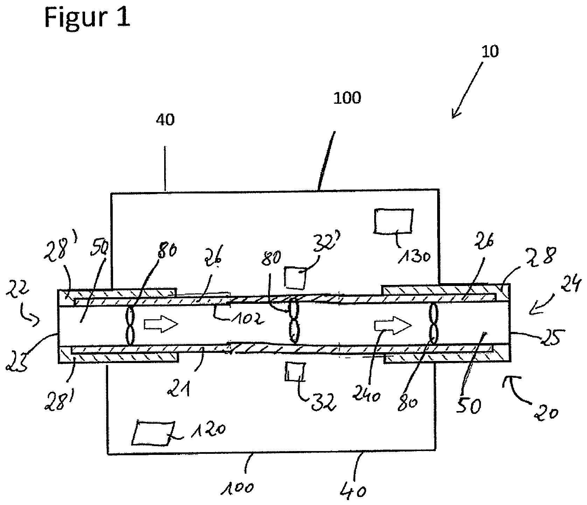

In Figur 1 ist schematisch eine Pumpvorrichtung 10 gezeigt, die ein Pumpgehäuse 20, in Form eines Rohres aufweist, sowie ein Bauteilgehäuse 40. Die Außenflächen 100 des Bauteilgehäuses 40 kommen insbesondere für eine implantierbare Pumpvorrichtung 10 mit dem Körper in Kontakt und sind daher bevorzugt biokompatibel ausgestaltet Das Pumpengehäuse 20 weist eine Wand 21 auf, die einen Innenbereich 50 umgibt. Die zum Innenbereich 50 weisende Fläche des Pumpengehäuses 20 wird als zugewandte Oberfläche 102 bezeichnet. Die zugewandte Oberfläche 102 kommt mit dem Fluid in Kontakt und ist daher insbesondere für eine implantierbare Pumpvorrichtung 10 bevorzugt biokompatibel ausgestaltet. In dem Innenbereich 50 des Pumpengehäuses 20 befindet sich mindestens ein Impeller 80, in diesem Fall befinden sich zwei Impeller 80 in dem Pumpengehäuse 20. Das Pumpengehäuse 20 weist einen ersten Hohlkörper 26 in der Mitte der Wand 21 auf. An dem ersten Ende 22, das gleichzeitig den Einlass 22 durch die Öffnung 23 definiert, weist die Wand 21 bzw. das Pumpengehäuse 20 einen ersten weiteren Hohlkörper 28 auf. Auf der gegenüberliegenden Seite des Pumpengehäuses 20 befindet sich das weitere Ende 24, in Form des Auslasses 24, beinhaltend die weitere Öffnung 25. Mittels des Impellers 80 kann ein Fluid in Pumprichtung 240 von dem Einlass 22 zum Auslass 24 gepumpt werden. Zwischen dem Bauteilgehäuse 40 und dem Pumpgehäuse befinden sich weitere Bauteile, wie eine Batterie 120 sowie eine Steuereinheit 130. Weiterhin befinden sich zwei Spulen 32 und 32' in dem Bauteilgehäuse 40. In diesem Beispiel weist das Pumpengehäuse 20 der Pumpvorrichtung 10 einen ersten Hohlkörper 26 und zwei weitere Hohlkörper 28, 28'auf. Das Pumpengehäuse 20 kann alternativ auch nur einen ersten Hohlkörper 26 und einen weiteren Hohlkörper 28 aufweisen, wie dies in den Figuren 3a bis 3e gezeigt ist.

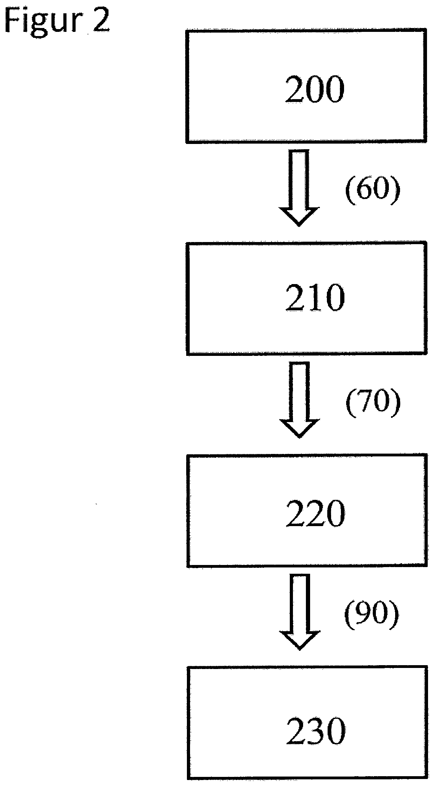

In Figur 2 ist schematisch der Ablauf des Verfahrens zur Herstellung eines Pumpengehäuses gezeigt. In dem Schritt a. bzw. a) 200 wird ein erstes Material 60 bereitgestellt. Das erste Material 60 ist beispielsweise eine Mischung aus mindestens zwei Pulvern. Das erste Material enthält die Zusammensetzung aus Beispiel 1, nämlich 20 Teile Aluminiumoxid (Al₂O₃) mit einer Korngröße von D₉₀ = 2 µm und ein Teil eines Bindemittels, in diesem Fall METAWAX P-50 erhältlich bei der Firma Zschimmer & Schwarz GmbH & Co. KG.

Das weitere Material 70 wird in Form einer Mischung, entsprechend Beispiel 2, aus 57 Gew.-% eines Platinpulvers mit einer Korngröße D₅₀ = 50 µm, und 38 Gew.-% Aluminiumoxid (Al₂O₃) mit einer Korngröße von D₉₀ = 2 µm sowie 5 Gew.-% eines Bindemittels, in diesem Fall METAWAX P-50 erhältlich bei der Firma Zschimmer & Schwarz GmbH & Co. KG in einem Schritt b. bzw. b) 210 ebenfalls bevorzugt in einem Behälter bereitgestellt. Der Behälter kann ein Metallbehälter mit einem Siebausgang sein. Bevorzugt weisen die Pulverkörner eine runde bis ovale Ausdehnung auf. Die Korngrößenangabe D₅₀ bedeutet, dass nicht mehr als 50 % der Teilchen größer sind als der angegebene Durchmesser. Die Korngrößenangabe D₉₀ bedeutet, dass nicht mehr als 90 % der Teilchen größer sind als der angegebene Durchmesser. Die Korngröße kann mit verschiedenen Methoden bestimmt werden. Bevorzugt wird die Korngröße mit Hilfe von Laserbeugung, Lichtmikroskopie, optische Einzelpartikelzählung oder einer Kombination mindestens zwei hiervon bestimmt. Weiterhin bevorzugt wird die Bestimmung der Korngröße so wie der Korngrößenverteilung anhand von optischer Einzelauswertung von Aufnahmen mittels Transmissions-Elektronen-Mikroskopie (TEM) vorgenommen. Außerdem kann die Korngröße dem Produktdatenblatt entnommen werden, das beim Rohstofflieferanten verfügbar und oftmals einer Lieferung beigepackt ist.

Das Kontaktieren in Schritt c. kann alternativ nach Formen des mindestens einen ersten Hohlkörpers 26 erfolgen oder bereits vor Formen des mindestens einen ersten Hohlkörpers 26. Das Formen ist in diesem Beispiel ein Sintern. Wird das Kontaktieren, wie in diesem Beispiel, nach dem Sintern beider Hohlkörper 26 und 28 durchgeführt, so wird in Schritt c. sowohl ein gesinterter Hohlkörper 26 als auch ein gesinterter Hohlkörper 28 miteinander kontaktiert. Das Kontaktieren findet in diesem Beispiel durch ein Erhitzen des weiteren Hohlkörpers 28 auf eine Temperatur von mindestens 250 °C in einem Umluft Ofen der Firma Heraeus Holding GmbH für 30 min und anschließendem in einander Schieben der beiden zylindrischen Hohlkörper 26 in Hohlkörper 28 statt. Der äußere weitere Hohlkörper 28 kühlt anschließend aus und schrumpft dabei. Auf diese Weise druckt sich die innere Fläche des weiteren Hohlkörpers 28 auf die äußere Oberfläche des ersten Hohlkörpers 26, sodass eine kraftschlüssige Verbindung zwischen den beiden Hohlkörpern 26 und 28 entsteht. Dieser Verbund 142 stellt in abgekühltem Zustand das Pumpengehäuse 20 dar.

Figuren 3a bis 3e zeigen verschiedene Möglichkeiten der Anordnung von dem ersten Hohlkörper 26 und dem weiteren Hohlkörper 28 in einem erfindungsgemäßen Pumpengehäuse 20. Das Pumpengehäuse 20 beinhaltet in seiner Wand 21 eine erste Öffnung 23 am Einlass 22 und eine weitere Öffnung 25 am Auslass 24. In der Figur 3a ist ein erster Hohlkörper 26 vollständig, also vom Einlass 22 bis zum Auslass 24 von dem weiteren Hohlkörper 28 umgeben.

In Figur 3b ist im Unterschied zu der Anordnung aus Figur 3a der erste Hohlkörper 26 auch an der Seite des Einlass 22 und des Auslasses 24 von dem weiteren Hohlkörper 28 umgeben. Die Innenfläche des Pumpengehäuses 20 weist sowohl Bereich des ersten Hohlkörpers 26 als auch Bereiche des weiteren Hohlkörpers 28 auf. Die gesamte äußere Fläche des Pumpengehäuses weist den weiteren Hohlkörper 28 auf. Weiterhin ist in dem Pumpengehäuse 20 ein Impeller 80 angeordnet, der ein Fluid in die gezeigte Pumprichtung 240 pumpen kann.

In Figur 3c ist im Unterschied zu der Anordnung aus Figur 3b nur am Einlass 22 der weitere Hohlkörper 28 um den ersten Hohlkörper geschoben. Auch hierein weist die gesamte äußere Fläche des Pumpengehäuses den weiteren Hohlkörper 28 auf.

Die Figur 3d weist wiederum, wie die Anordnung des Pumpengehäuses 20 aus Figur 3a über den gesamten Innenbereich 50 den ersten Hohlkörper 26 auf. Das bedeutet, dass die gesamte Innenfläche 140 durch die weitere Oberfläche gebildet ist. An der Außenseite des Pumpengehäuses 20 befindet sich sowohl ein Teil des ersten Hohlkörpers 26 am Auslass 24 als auch ein Teil des weiteren Hohlkörpers 28 am Einlass 22. Der Einlass beinhaltet folglich sowohl den ersten 26 als auch den weiteren Hohlkörper 28.

Die Figur 3e weist am Einlass 22 und am Auslass 24 nur den ersten Hohlkörper 26 auf. Der weitere Hohlkörper 28 ist nur über einen mittleren Teil des Pumpengehäuses 20 über den ersten Hohlkörper 26 geschoben. Die Innenfläche 140 diese Pumpengehäuses 20 weist nur den ersten Hohlkörper 26 auf.

### Bezugszeichenliste

- 10: Pumpvorrichtung
- 20: Gehäuse, Pumpengehäuse
- 21: Wand
- 22: erstes Ende / Einlass
- 23: erste Öffnung
- 24: weiteres Ende /Auslass
- 25: weitere Öffnung
- 26: erster Hohlkörper
- 28, 28': weiterer Hohlkörper
- 32, 32': Spule
- 40: Bauteilgehäuse
- 50: Innenbereich
- 60: erstes Material
- 70: weiteres Material
- 80: Impeller
- 90: Vorläufer/Pumpengehäusevorläufer
- 100: Außenfläche
- 110: elektrisches Bauteil
- 120: Batterie
- 130: Steuereinheit
- 140: weitere Oberfläche/Innenfläche
- 142: Verbund
- 200: Schritt a. / Schritt a)
- 210: Schritt b. / Schritt b)
- 220: Schritt c. / Schritt c)
- 240: Pumprichtung

## Patentansprüche

1. Eine Pumpvorrichtung (10), beinhaltend:
i. einen Impeller (80);
ii. ein Pumpengehäuse (20), das einen Innenbereich (50) zumindest zu einem Teil umgibt, mit einem Einlass (22) und einem Auslass (24);
wobei der Impeller (80) im Innenbereich (50) des Pumpengehäuses vorgesehen (20) ist,
wobei das Pumpengehäuse (20) einen Verbund (142) aus einem ersten Hohlköper (26) und einem den ersten Hohlkörper (26) auf der dem Innenbereich (50) abgewandten Seite zumindest zum Teil umgebenden weiteren Hohlkörper (28, 28') beinhaltet;
wobei mindestens 60 % der Oberfläche der dem Innenraum (50) abgewandten Seite des ersten Hohlkörpers (26) mit dem weiteren Hohlkörper (28, 28') verbunden sind, bezogen auf die Oberfläche der dem Innenraum (50) abgewandten Seite des ersten Hohlkörpers (26),
wobei das Pumpengehäuse (20) mit einer weiteren Oberfläche (140) des ersten Hohlkörpers (26) dem Innenbereich (50) zugewandt ist;
wobei die dem Innenbereich (50) des Pumpengehäuses (20) zugewandte weitere Oberfläche (140) des ersten Hohlkörpers (26) eine Härte von mindestens 330 HV aufweist,
wobei der weitere Hohlkörper (28, 28') einen Metallgehalt von mindestens 50 Gew.-%, bezogen auf die Gesamtmasse des weiteren Hohlkörpers (28), beinhaltet,
wobei die Pumpvorrichtung dazu geeignet ist, in den Körper eines Menschen oder eines Tieres eingebracht zu werden.

2. Die Pumpvorrichtung (10) nach Anspruch 1, wobei der erste Hohlkörper (26) und der weitere Hohlkörper (28, 28') durch einen Kraftschluss verbunden sind.

3. Die Pumpvorrichtung (10) nach Anspruch 1 oder 2, wobei der erste Hohlkörper (26), oder der weitere Hohlkörper (28, 28') oder beide Hohlkörper (26, 28), jeweils einen zylindrischen Hohlkörper bilden.

4. Die Pumpvorrichtung (10) nach einem der vorhergehenden Ansprüche, wobei das Material des ersten Hohlkörpers (26) eine Keramik ist, bevorzugt ausgewählt aus der Gruppe bestehend aus Aluminiumoxid (Al₂O₃), Zirkoniumdioxid (ZrO₂), Aluminiumoxid verstärktes Zirkoniumoxid (ZTA), Zirkoniumoxid verstärktes Aluminiumoxid, Yttrium verstärktes Zirkoniumoxid (Y-TZP), Aluminiumnitrid (AIN), Magnesiumoxid (MgO), Piezokeramik, Barium(Zr, Ti)oxid, Barium(Ce, Ti)oxid, Titannitrid (TiN), Siliziumdioxid (SiO₂) und Natrium-Kalium-Niobat oder einer Mischung aus mindestens zwei hiervon.

5. Die Pumpvorrichtung (10) nach einem der vorhergehenden Ansprüche, wobei das Metall des weiteren Hohlkörpers (28, 28') ausgewählt ist aus der Gruppe bestehend aus Platin (Pt), Eisen (Fe), Edelstahl, Iridium (Ir), Niob (Nb), einer Niob-Legierung, Molybdän (Mo), Wolfram (W), Titan (Ti), einer Titan-Legierung, Kobalt (Co), Chrom (Cr), einer Kobalt-Chrom-Legierung, Tantal (Ta) und Zirkonium (Zr) oder einer Mischung aus mindestens zwei hiervon.

6. Die Pumpvorrichtung (10) nach einem der vorhergehenden Ansprüche, wobei der erste Hohlkörper (26) weniger als 10 Gew.-%, bezogen auf die Gesamtmasse des ersten Hohlkörpers (26), an Metall beinhaltet.

7. Die Pumpvorrichtung (10) nach einem der vorhergehenden Ansprüche, wobei die Pumpvorrichtung zumindest zu einem Teil von einem Bauteilgehäuse (40) umgeben ist, wobei der Verbund (142) der Pumpvorrichtung (10) mit dem Bauteilgehäuse (40) verbunden ist.

8. Die Pumpvorrichtung (10) nach einem der vorhergehenden Ansprüche, wobei das Pumpengehäuse (20) ein Volumen in einem Bereich von 0,1 cm³ bis 10 cm³ aufweist.

9. Die Pumpvorrichtung (10) nach einem der beiden vorhergehenden Ansprüche, wobei das Bauteilgehäuse (40) mindestens 30 Gew.-%, bezogen auf die Gesamtmasse des Bauteilgehäuses (40), Titan beinhaltet.

10. Die Pumpvorrichtung (10) nach einem der vorhergehenden Ansprüche, wobei der erste Hohlkörper (26) eine magnetische Permeabilität von weniger als 2 µ aufweist.

11. Ein Verfahren zur Herstellung eines Pumpengehäuses (20) für eine Pumpvorrichtung (10) beinhaltend die Schritte:
a. Bereitstellen eines ersten Hohlkörpers (26);
b. Bereitstellen eines weiteren Hohlkörpers (28, 28');
wobei mindestens der erste Hohlkörper (26) als zylindrischer Hohlkörper (20) ausgebildet ist;
c. Kontaktieren des ersten Hohlkörpers (26) mit dem weiteren Hohlkörper (28', 28'),
wobei ein Verbund (142) gebildet wird,
wobei das Kontaktieren als kraftschlüssiges oder stoffschlüssiges Verbinden zwischen dem ersten Hohlkörper (26) und dem weiteren Hohlkörper (28, 28') ausgeführt wird;
wobei die Pumpvorrichtung dazu geeignet ist, in den Körper eines Menschen oder eines Tieres eingebracht zu werden.

12. Das Verfahren nach dem vorhergehenden Anspruch, wobei das Kontaktieren in Schritt c. ausgewählt ist aus der Gruppe bestehend aus einem Kleben, einem Pressen, einem Sintern oder einer Kombination aus mindestens zwei davon.

13. Ein Pumpengehäuse erhältlich nach einem Verfahren gemäß Anspruch 11 oder 12.

14. Ein Gehäuse (20), das einen Innenbereich (50) zumindest zu einem Teil umgibt, mit einem Einlass (22) und einem Auslass (24);
wobei das Gehäuse (20) einen Verbund (142) aus einem ersten Hohlköper (26) und
einem den ersten Hohlkörper (26) auf der dem Innenbereich (50) abgewandten Seite zumindest zum Teil umgebenden weiteren Hohlkörper (28, 28') beinhaltet;
wobei mindestens 60 % der Oberfläche der dem Innenraum (50) abgewandten Seite des ersten Hohlkörpers (26) mit dem weiteren Hohlkörper (28, 28') verbunden sind, bezogen auf die Oberfläche der dem Innenraum (50) abgewandten Seite des ersten Hohlkörpers (26),
wobei das Gehäuse (20) mit einer weiteren Oberfläche (140) des ersten Hohlkörpers (26) dem Innenbereich (50) zugewandt ist;
wobei die dem Innenbereich (50) des Gehäuses (20) zugewandte weitere Oberfläche (140) des ersten Hohlkörpers (26) eine Härte von mindestens 330 HV aufweist,
wobei der weitere Hohlkörper (28, 28') einen Metallgehalt von mindestens 50 Gew.-%, bezogen auf die Gesamtmasse des weiteren Hohlkörpers (28), beinhaltet;
wobei das Gehäuse (20) dazu geeignet ist, in den Körper eines Menschen oder eines Tieres eingebracht zu werden.

15. Eine Pumpvorrichtung (10) beinhaltend mindestens ein Gehäuse (20) nach Anspruch 14 oder ein Gehäuse (20) erhältlich nach einem Verfahren gemäß mindestens einem der Ansprüche 11 oder 12.

## Claims

1. A pumping device (10), including:
i. an impeller (80);
ii. a pump housing (20), which surrounds an inner area (50) at least partially, with an inlet (22) and an outlet (24);
wherein the impeller (80) is provided (20) in the inner region (50) of the pump housing,
wherein the pump housing (20) comprises a composite (142) of a first hollow body (26) and a further hollow body (28, 28') at least partially surrounding the first hollow body (26) on the side facing away from the inner region (50);
wherein at least 60% of the surface of the side of the first hollow body (26) remote from the interior space (50) is connected to the further hollow body (28, 28'), relative to the surface of the side of the first hollow body (26) remote from the interior space (50),
wherein the pump housing (20) faces the inner region (50) with a further surface (140) of the first hollow body (26);
wherein the further surface (140) of the first hollow body (26) facing the inner region (50) of the pump housing (20) has a hardness of at least 330 HV,
wherein the further hollow body (28, 28') contains a metal content of at least 50% by weight, based on the total mass of the further hollow body (28),
wherein the pumping device is suitable to be introduced into the body of a human or an animal.

2. The pumping device (10) according to claim 1, wherein the first hollow body (26) and the further hollow body (28, 28') are connected by a frictional connection.

3. The pump device (10) according to claim 1 or 2, wherein the first hollow body (26), or the further hollow body (28, 28'), or both hollow bodies (26, 28), each form a cylindrical hollow body.

4. The pumping device (10) according to any of the preceding claims, wherein the material of the first hollow body (26) is a ceramic, preferably selected from the group consisting of alumina (Al₂O₃), zirconia (ZrO₂), alumina reinforced zirconia (ZTA), Zirconia reinforced alumina, yttria reinforced zirconia (Y-TZP), aluminium nitride (AIN), magnesium oxide (MgO), piezoceramics, barium (Zr, Ti) oxide, barium (Ce, Ti) oxide, titanium nitride (TiN), silicon dioxide (SiO₂) and sodium potassium niobate or a mixture of at least two of them.

5. The pump device (10) according to any of the preceding claims, wherein the metal of the further hollow body (28, 28') is selected from the group consisting of platinum (Pt), iron (Fe), stainless steel, iridium (Ir), niobium (Nb), a niobium alloy, molybdenum (Mo), tungsten (W), titanium (Ti), a titanium alloy, cobalt (Co), chromium (Cr), a cobalt-chromium alloy, tantalum (Ta) and zirconium (Zr) or a mixture of at least two thereof.

6. The pump device (10) according to any of the foregoing claims, wherein the first hollow body (26) contains less than 10% by weight, based on the total mass of the first hollow body (26), of metal.

7. The pumping device (10) according to any of the foregoing claims, the pumping device being at least partially surrounded by a component housing (40), the composite (142) of the pumping device (10) being connected to the component housing (40).

8. The pumping device (10) according to any of the foregoing claims, wherein the pump housing (20) has a volume in a range of 0.1 cm³ to 10 cm³.

9. The pumping device (10) according to either of the previous two claims, wherein the component housing (40) contains at least 30% by weight, based on the total mass of the component housing (40), of titanium.

10. The pump device (10) according to any of the foregoing claims, wherein the first hollow body (26) has a magnetic permeability of less than 2 µ.

11. A method of manufacturing a pump housing (20) for a pumping device (10) comprising the steps of
a. Provision of a first hollow body (26);
b. Provision of a further hollow body (28, 28');
wherein at least the first hollow body (26) is formed as a cylindrical hollow body (20);
c. Contact the first hollow body (26) with the further hollow body (28, 28'),
whereby a composite (142) is formed,
wherein the contacting is carried out as a non-positive or material-locking connection between the first hollow body (26) and the further hollow body (28, 28');
wherein the pumping device is suitable to be introduced into the body of a human or an animal.

12. The method according to the preceding claim, wherein the contacting in step c. is selected from the group consisting of bonding, pressing, sintering or a combination of at least two of them.

13. A pump casing obtainable by a method according to claim 11 or 12.

14. A housing (20) at least partially surrounding an interior (50) with an inlet (22) and an outlet (24);
wherein the housing (20) comprises a composite (142) of a first hollow body (26) and a further hollow body (28, 28') at least partially surrounding the first hollow body (26) on the side facing away from the inner region (50);
wherein at least 60% of the surface of the side of the first hollow body (26) remote from the interior space (50) is connected to the further hollow body (28, 28'), relative to the surface of the side of the first hollow body (26) remote from the interior space (50),
wherein the housing (20) faces the inner region (50) with a further surface (140) of the first hollow body (26);
wherein the further surface (140) of the first hollow body (26) facing the inner region (50) of the housing (20) has a hardness of at least 330 HV,
wherein the further hollow body (28, 28') contains a metal content of at least 50% by weight, based on the total mass of the further hollow body (28);
wherein the housing (20) is suitable for being inserted into the body of a human or animal.

15. A pump device (10) comprising at least one housing (20) according to claim 14 or a housing (20) obtainable by a method according to at least one of claims 11 or 12.

## Revendications

1. Un dispositif de pompage (10), comprenant :
i. une roue (80) ;
ii. un logement de roue (20), qui entoure au moins partiellement une zone intérieure (50), avec une entrée (22) et une sortie (24) ;
dans lequel la roue (80) est prévue (20) dans la zone intérieure (50) du corps de pompe,
dans lequel le logement de roue (20) comprend un composite (142) d'un premier corps creux (26) et d'un autre corps creux (28, 28') entourant au moins partiellement le premier corps creux (26) sur le côté opposé à la zone intérieure (50) ;
dans lequel au moins 60 % de la surface du côté du premier corps creux (26) opposé à l'espace intérieur (50) est reliée à l'autre corps creux (28, 28'), par rapport à la surface du côté du premier corps creux (26) opposé à l'espace intérieur (50),
dans lequel le logement de roue(20) est tourné vers la zone intérieure (50) avec une autre surface (140) du premier corps creux (26) ;
dans lequel l'autre surface (140) du premier corps creux (26) tournée vers la zone intérieure (50) du corps de pompe (20) présente une dureté d'au moins 330 HV,
dans lequel l'autre corps creux (28, 28') contient une teneur en métal d'au moins 50% en poids, par rapport à la masse totale de l'autre corps creux (28),
dans lequel le dispositif de pompage est approprié pour être introduit dans le corps d'un humain ou d'un animal.

2. Dispositif de pompage (10) selon la revendication 1, dans lequel le premier corps creux (26) et l'autre corps creux (28, 28') sont reliés par une liaison à friction.

3. Dispositif de pompe (10) selon la revendication 1 ou 2, dans lequel le premier corps creux (26), ou l'autre corps creux (28, 28'), ou les deux corps creux (26, 28), forment chacun un corps creux cylindrique.

4. Dispositif de pompage (10) selon l'une quelconque des revendications précédentes, dans lequel le matériau du premier corps creux (26) est une céramique, de préférence choisie dans le groupe constitué par l'oxide de l'aluminium (Al₂O₃), l'oxide de zirconium (ZrO₂), l'oxide de zirconium renforcée par l'oxide de aluminium (ZTA), l'oxide de aluminium renforcée par l'oxide de zirconium, l'oxide de zirconium renforcée par yttrium (Y-TZP), nitrure d'aluminium (AIN), oxyde de magnésium (MgO), piézocéramique, oxyde de baryum (Zr, Ti), oxyde de baryum (Ce, Ti), nitrure de titane (TiN), dioxyde de silicium (SiO₂) et niobate de sodium et de potassium ou un mélange d'au moins deux d'entre eux.

5. Dispositif de pompage (10) selon l'une des revendications précédentes, dans lequel le métal de l'autre corps creux (28, 28') est choisi dans le groupe constitué par le platine (Pt), le fer (Fe), l'acier inoxydable, l'iridium (Ir), le niobium (Nb), un alliage de niobium, de molybdène (Mo), de tungstène (W), de titane (Ti), un alliage de titane, de cobalt (Co), de chrome (Cr), un alliage de cobalt-chrome, de tantale (Ta) et de zirconium (Zr) ou un mélange d'au moins deux de ceux-ci.

6. Dispositif de pompage (10) selon l'une quelconque des revendications précédentes, dans lequel le premier corps creux (26) contient moins de 10 % en poids, par rapport à la masse totale du premier corps creux (26), de métal.

7. Le dispositif de pompage (10) selon l'une des revendications précédentes, le dispositif de pompage étant au moins partiellement entouré par un boîtier de composant (40), le composite (142) du dispositif de pompage (10) étant relié au boîtier de composant (40).

8. Dispositif de pompage (10) selon l'une quelconque des revendications précédentes, dans lequel le boîtier de pompe (20) a un volume dans une plage de 0,1 cm³ à 10 cm.

9. Dispositif de pompage (10) selon l'une ou l'autre des deux revendications précédentes, dans lequel le boîtier de composant (40) contient au moins 30% en poids, par rapport à la masse totale du boîtier de composant (40), de titane.

10. Dispositif de pompage (10) selon l'une des revendications précédentes, dans lequel le premier corps creux (26) présente une perméabilité magnétique inférieure à 2 µ.

11. Procédé de fabrication d'un logement de roue (20) pour un dispositif de pompage (10) comprenant les étapes suivantes
a. Mise à disposition d'un premier corps creux (26) ;
b. Mise à disposition d'un autre corps creux (28, 28') ; dans lequel au moins le premier corps creux (26) est réalisé sous la forme d'un corps creux cylindrique (20) ;
c. Mettre en contact le premier corps creux (26) avec l'autre corps creux (28', 28'),
par lequel un composite (142) est formé,
dans lequel la mise en contact s'effectue sous la forme d'une liaison par force ou par matière entre le premier corps creux (26) et l'autre corps creux (28, 28') ;
dans lequel le dispositif de pompage est approprié pour être introduit dans le corps d'un humain ou d'un animal.

12. Procédé selon la revendication précédente, dans lequel la mise en contact à l'étape c. est choisie dans le groupe constitué par la liaison, le pressage, le frittage ou une combinaison d'au moins deux d'entre eux.

13. Corps de pompe pouvant être obtenu par un procédé selon la revendication 11 ou 12.

14. Boîtier (20) entourant au moins partiellement un intérieur (50) avec une entrée (22) et une sortie (24) ;
dans lequel le boîtier (20) comprend un composite (142) d'un premier corps creux (26) et d'un autre corps creux (28, 28') entourant au moins partiellement le premier corps creux (26) sur le côté opposé à la zone intérieure (50) ;
dans lequel au moins 60 % de la surface du côté du premier corps creux (26) opposé à l'espace intérieur (50) est reliée à l'autre corps creux (28, 28'), par rapport à la surface du côté du premier corps creux (26) opposé à l'espace intérieur (50),
dans lequel le boîtier (20) est tourné vers la zone intérieure (50) avec une autre surface (140) du premier corps creux (26) ;
dans lequel l'autre surface (140) du premier corps creux (26) tournée vers la zone intérieure (50) du boîtier (20) présente une dureté d'au moins 330 HV,
dans lequel l'autre corps creux (28, 28') contient une teneur en métal d'au moins 50% en poids, par rapport à la masse totale de l'autre corps creux (28) ;
dans lequel le boîtier (20) est approprié pour être inséré dans le corps d'un humain ou d'un animal.

15. Dispositif de pompe (10) comprenant au moins un boîtier (20) selon la revendication 14 ou un boîtier (20) pouvant être obtenu par un procédé selon au moins l'une des revendications 11 ou 12.
